# EUROPÄISCHE PATENTSCHRIFT

(11) **EP 2 895 198 B1**
(45) Veröffentlichungstag und Bekanntmachung des Hinweises auf die Patenterteilung: **04.07.2018**
(21) Anmeldenummer: 13783244.0
(22) Anmeldetag: 13.09.2013
(51) Int. Cl.: A61K 47/69, C12N 5/00

(54) **NICHTKOVALENTE SELBSTORGANISIERENDE HYDROGELMATRIX FÜR BIOTECHNOLOGISCHE ANWENDUNGEN**
NON-COVALENT SELFORGANISING HYDROGELMATRIX FOR BIOTECHNOLIGICAL APPLICATIONS
MATRICE D'HYDROGEL NON-COVALENT AUTO-ORGANISANT, POUR DES APPLICATIONS EN BIOTECHNOLOGIE

(30) Priorität: 13.09.2012 DE 102012108560
(43) Veröffentlichungstag der Anmeldung: 22.07.2015
(73) Patentinhaber: Technische Universität Dresden, 01069 Dresden (DE); Leibniz-Institut für Polymerforschung Dresden e.V., 01069 Dresden (DE)
(72) Erfinder: WIEDUWILD, Robert, 01069 Dresden (DE); ZHANG, Yixin, 01219 Dresden (DE); WERNER, Carsten, 01069 Dresden (DE); TSURKAN, Mikhail, 01217 Dresden (DE); FREUDENBERG, Uwe, 01328 Dresden (DE)
(74) Vertreter: Sperling, Thomas
(86) Internationale Anmeldenummer: PCT/DE2013/100327
(87) Internationale Veröffentlichungsnummer: WO 2014/040591

(56) Entgegenhaltungen:
- WO-A1-94/04176
- WO-A2-2008/124165
- US-B1- 6 958 212
- NORI YAMAGUCHI ET AL: "Rheological Characterization of Polysaccharide-Poly(ethylene glycol) Star Copolymer Hydrogels", BIOMACROMOLECULES, Bd. 6, Nr. 4, 28. Mai 2005 (2005-05-28), Seiten 1931-1940, XP055102352, ISSN: 1525-7797, DOI: 10.1021/bm0500042 in der Anmeldung erwähnt
- YAMAGUCHI N ET AL: "Polysaccharide-poly(ethylene glycol) star copolymer as a scaffold for the production of bioactive hydrogels", BIOMACROMOLECULES, Bd. 6, Nr. 4, Juli 2005 (2005-07), Seiten 1921-1930, XP002539059, ISSN: 1525-7797, DOI: 10.1021/BM050003 [gefunden am 2005-05-28] in der Anmeldung erwähnt
- KYUNG JAE JEONG ET AL: "Interplay between Covalent and Physical Interactions within Environment Sensitive Hydrogels", BIOMACROMOLECULES, Bd. 10, Nr. 5, 23. März 2009 (2009-03-23), Seiten 1090-1099, XP055102304, ISSN: 1525-7797, DOI: 10.1021/bm801270k
- MIKHAIL V. TSURKAN ET AL: "Enzymatically degradable heparin-polyethylene glycol gels with controlled mechanical properties", CHEMICAL COMMUNICATIONS, Bd. 46, Nr. 7, 16. Dezember 2009 (2009-12-16), Seiten 1141-1143, XP055102315, ISSN: 1359-7345, DOI: 10.1039/b921616b in der Anmeldung erwähnt
- MIKHAIL V. TSURKAN ET AL: "Modular StarPEG-Heparin Gels with Bifunctional Peptide Linkers", MACROMOLECULAR RAPID COMMUNICATIONS, Bd. 31, Nr. 17, 16. August 2010 (2010-08-16), Seiten 1529-1533, XP055102320, ISSN: 1022-1336, DOI: 10.1002/marc.201000155 in der Anmeldung erwähnt
- SEAL B L ET AL: "Physical matrices stabilized by enzymatically sensitive covalent crosslinks", ACTA BIOMATERIALIA, Bd. 2, Nr. 3, 1. Mai 2006 (2006-05-01), Seiten 241-251, XP028009579, ISSN: 1742-7061, DOI: 10.1016/J.ACTBIO.2005.12.008 [gefunden am 2006-05-01]
- ALISON B. PRATT ET AL: "Synthetic extracellular matrices for in situ tissue engineering", BIOTECHNOLOGY AND BIOENGINEERING, Bd. 86, Nr. 1, 12. Februar 2004 (2004-02-12), Seiten 27-36, XP055102381, ISSN: 0006-3592, DOI: 10.1002/bit.10897
- BRANDON L. SEAL ET AL: "Physical Polymer Matrices Based on Affinity Interactions between Peptides and Polysaccharides", BIOMACROMOLECULES, Bd. 4, Nr. 6, 1. November 2003 (2003-11-01), Seiten 1572-1582, XP055102384, ISSN: 1525-7797, DOI: 10.1021/bm0342032
- FREUDENBERG U ET AL: "A star-PEG-heparin hydrogel platform to aid cell replacement therapies for neurodegenerative diseases", BIOMATERIALS, Bd. 30, Nr. 28, 1. Oktober 2009 (2009-10-01), Seiten 5049-5060, XP026420872, ISSN: 0142-9612 [gefunden am 2009-06-27] in der Anmeldung erwähnt
- NIE T ET AL: "Production of heparin-containing hydrogels for modulating cell responses", ACTA BIOMATERIALIA, Bd. 5, Nr. 3, März 2009 (2009-03), Seiten 865-875, XP025990683, ISSN: 1742-7061, DOI: 10.1016/J.ACTBIO.2008.12.004 [gefunden am 2009-02-25]
- BENOIT ET AL: "The effect of heparin-functionalized PEG hydrogels on three-dimensional human mesenchymal stem cell osteogenic differentiation", BIOMATERIALS, Bd. 28, Nr. 1, 1. Januar 2007 (2007-01-01) , Seiten 66-77, XP005666653, ISSN: 0142-9612, DOI: 10.1016/J.BIOMATERIALS.2006.08.033
- BRANDON L. SEAL ET AL: "Viscoelastic Behavior of Environmentally Sensitive Biomimetic Polymer Matrices", MACROMOLECULES, Bd. 39, Nr. 6, 23. Februar 2006 (2006-02-23), Seiten 2268-2274, XP055102566, ISSN: 0024-9297, DOI: 10.1021/ma0524528

## Beschreibung

Die Erfindung betrifft eine nichtkovalente selbstorganisierende Hydrogelmatrix für biotechnologische Anwendungen. Des Weiteren betrifft die Erfindung aus dieser Hydrogelmatrix gebildete Hydrogelmatrixkugeln sowie einen Verbund der Hydrogelmatrix oder der Hydrogelmatrixkugeln mit darin eingebetteten Zellen. Andere Aspekte der Erfindung betreffen eine Kapsel zur gezielten Freisetzung von therapeutischen Reagenzien, eine Zusammensetzung aus der Hydrogelmatrix, therapeutischen Chemikalien und Reagenzien sowie ein Hybridsystem aus einer nichtkugelförmigen Hydrogelmatrix und aus Hydrogelmatrixkugeln.

Das Design und die Synthese von sich selbstorganisierenden Makromolekül-Systemen für Anwendungen im Bereich "Life-Science" und darüber hinaus ist von großem Interesse für Chemie, Materialwissenschaften und Biomedizin. Verschiedene Hydrogele haben große Hoffnungen in eine Anwendbarkeit für den biomedizinischen Bereich geweckt, wie zum Beispiel im Bereich des Wirkstofftransports und der Gewebezüchtung. Eine Reihe von Polymer-Matrices, die aus lebenden Quellen stammen, wie zum Beispiel Matrigel und Kollagen-Hydrogele, haben sich bezüglich ihrer hohen Biokompatibilität in der Zellkultur allen vorhandenen synthetischen Polymeren überlegen gezeigt. Allerdings haben solche Biomaterialien aus lebenden Quellen keine definierte chemische Zusammensetzung, was ihre breite Anwendung in der Biomedizin verhindert.

Andererseits kann ein System von polymeren Matrices mit hoher Haltbarkeit zu einer großen Vielfalt von "maßgeschneiderten" Strukturen führen, die für unterschiedliche Anwendungen geeignet sind. Auf diese Weise können viele Eigenschaften des Systems beeinflusst werden. Beispielhaft sind an dieser Stelle verschiedene physikalische und biochemische Eigenschaften für das Kultivieren verschiedener Zelltypen oder unterschiedliche Gelierungzeiten und Abbaugeschwindigkeiten für eine Implantation an verschiedenen erkrankten Stellen genannt. Letztlich sollte ein ideales synthetisches Hydrogelsystem nicht nur in der Lage sein, die biologischen Funktionen von verschiedenen extrazellulären Matrices (ECM) zu imitieren, sondern auch die Möglichkeit bieten, diese Funktionen zu steuern und zu optimieren. Die Gestaltung von solchen abstimmbaren Materialien für biomedizinische Anwendungen stellt eine große Herausforderung dar. Insbesondere gilt das für biologische Untersuchungen über die Zusammensetzung und die Funktionen der verschiedenen extrazellulären Matrices (ECM), für Polymer- und materialwissenschaftliche Untersuchungen zum Zwecke der Rekonstruktion beziehungsweise zur Konstruktion solcher Systeme sowie für chemische Untersuchungen über die Steuerung der Prozesse.

Synthetische Polymere, wie Polyethylenglykol (PEG), Polyvinylalkohol, Poly(N-isoproprylacrylamid), Poly(milchsäure-co-Glykolsäure) (PLGA) und Copolymere von diesen und anderen Polymeren, stellen viele nützliche Systeme für den Einsatz im biomedizinischen Bereich bereit. Einerseits sind die Polymere als chemische Strukturen mit minimaler Wechselwirkung mit biologischen Systemen entwickelt worden. Beispielsweise werden sie vom Immunsystem oft nicht als Antigene erkannt, wodurch Komplikationen der Immunogenität vermieden werden. Doch andererseits führt dieser Vorteil des Mangels an Funktion auch zu einer mangelnden Ähnlichkeit dieser Polymere mit wichtigen Funktionen des lebenden Systems, vor allem hinsichtlich der Dynamik und der Signalisierung in der extrazellulären Matrix (ECM).

Das Konjugieren von Bio-Makromolekülen und synthetischen Polymeren stellt einen interessanten Weg dar, um die oben genannten Hydrogelsysteme zu entwerfen. Die Bio-Makromoleküle der Wahl können entweder synthetischen Ursprungs, wie zum Beispiel Peptide aus der Festphasenpeptidsynthese und DNA aus der Festphasen-Oligonukleotid-Synthese, oder biologischen Ursprungs mit gut definierter chemischer Zusammensetzung sein. Sehr wichtig ist, dass die Bio-Makromoleküle keine toxischen Eigenschaften und eine niedrige Immunogenität aufweisen. Sowohl kovalente als auch nichtkovalente Methoden zur Vernetzung können verwendet werden, wobei die nichtkovalenten Methoden wegen der Möglichkeit, verschiedene Gele herzustellen, von besonderem Forschungsinteresse sind. Darüber hinaus werden es nichtkovalente, sich selbstorganisierende Systeme ermöglichen, Zellen in ein Matrix-System einzubetten, ohne dass dies mit chemischen Reaktionen verbunden ist.

Der Einbau von Polysaccharid-Molekülen in Biohybridmaterialien wird zunehmend angewendet, um natürliche ECM-Funktionen in synthetischen oder halbsynthetischen Materialien zu erreichen. Insbesondere wurden Hyaluronsäure und Heparin in einer Reihe von Designkonzepten aufgrund ihrer biologischen Aktivitäten sowie ihrer biologischen Verfügbarkeit verwendet. Heparin, ein Glycosaminoglycan (GAG) mit der höchsten anionischen Ladungsdichte, die in einem Biopolymer auftritt, wird aufgrund seiner Affinität für eine Vielzahl von wichtigen Signalmolekülen verwendet. Während Heparin ein komplexes Polymer ist, das aus einer biologischen Quelle extrahiert werden kann und entsprechende Proben sich voneinander bezüglich der Massenverteilung, der Zusammensetzung von Zuckermonomeren sowie dem Sulfatierungsgrad unterscheiden, sind Dextransulfat und Cyclodextrinsulfat einfachere Oligosaccharide. Außerdem können einige Cyclodextrinsulfat-Verbindungen als reine chemische Verbindungen erhalten werden.

Die Entwicklung von biokompatiblem Hydrogel stellt einen interessanten Ansatz für Forschungen sowohl auf dem Gebiet der Materialwissenschaften als auch auf dem Gebiet der Biomedizin dar. Viele nichtkovalente selbstorganisierende Hydrogele oder Oligosaccharid enthaltende Hydrogele wurden in den letzten zehn Jahren entwickelt. Bei **Kiicks et al.** (N. Yamaguchi, B.-S. Chae, L. Zhang, K. L. Kiick, E. M. Furst, Biomacromolecules 2005, 6, 1931-1940; N. Yamaguchi, K. L. Kiick, Biomacromolecules 2005, 6, 1921-1930; L. Zhang, E. M. Furst, K. L. Kiick, Journal of Controlled Release 2006, 114, 130-142; K. L. Kiick, Soft Matter 2008, 4, 29-37; F. J. Spinelli, K. L. Kiick, E. M. Furst, Biomaterials 2008, 29, 1299-1306) sind die Verwendung von niedermolekularem Heparin-Stern-PEG-Konjugat, das heißt von an vierarmigem Stern-PEG gekoppelten Heparin (NMH), und die Verwendung von Peptid-Stern-PEG-Konjugat, das heißt von an vierarmigem Stern-PEG gekoppelten natürlichen Derivaten von Peptiden, beschrieben. Nach dem Mischen dieser beiden Verbindungen, nämlich Heparin-Stern-PEG und Peptid-Stern-PEG, wird ein Hydrogel nichtkovalent ausgebildet. Die Fähigkeit von Heparin mit niedrigem Molekulargewicht (LMWH), multiple Partner zu binden, wurde zum Anbringen und zur Abgabe von Wachstumsfaktoren oder anderen gewünschten Heparin-bindenden Peptiden beziehungsweise Proteinen an die nichtkovalent angeordneten Matrices ausgenutzt. So wurde auch die Anordnung dieser Hydrogele mit dem dimeren Heparin-bindenden Wachstumsfaktor VEGF (VEGF = vascular endothelial growth factor) genutzt. Ein interessantes Ergebnis der mit einem Wachstumsfaktor vermittelten Hydrogelnetzwerke ist die Fähigkeit zu einer entsprechenden Rezeptorvermittelten Gel-Erosion. VEGF-Vernetzungen können in Gegenwart von VEGF-Rezeptoren, welche die Proliferation und Migration von vaskulären Endothelzellen steuern, selektiv konkurrieren und dissoziieren.

Bei der Entwicklung und Synthese von Hydrogelen werden zunehmend bioorthogonale Reaktionen und photoinduzierte Thiol-En-Reaktionen angewendet. Die sogenannte Klick-Chemie ermöglicht sehr selektive und orthogonale Reaktionen, die mit hoher Effizienz und unter einer Reihe von milden Bedingungen ablaufen. **Anseth et al.** (S. B. Anderson, C.-C. Lin, D. V. Kuntzler, K. S. Anseth, Biomaterials 2011, 32, 3564-3574) haben eine sicher funktionierende Synthesestrategie eingeführt, in der makromolekulare Vorstufen mittels einer Kupfer-Klick-Chemie reagieren, was die direkte Einkapselung von Zellen innerhalb von Klick-Hydrogelen erlaubt. Die milde chemische Reaktion zwischen Thiol und Vinylsulfon wurde auch intensiv zur Erzeugung verschiedener Hydrogele verwendet. In jüngster Zeit hat eine Synergie dieser chemischen und biochemischen Reaktionen zum Entwurf und zur Synthese einer Reihe von multifunktionalisierten Hydrogelsystemen geführt.

Es wurde in eigenen Arbeiten der Erfinder ein modulares System biohybrider Hydrogele auf der Basis von kovalent vernetztem Heparin und Stern-PEG entwickelt (A. Zieris, S. Prokoph, P. Welzel, M. Grimmer, K. Levental, W. Panyanuwat, U. Freudenberg, C. Werner, Journal of Materials Science: Materials in Medicine 2010, 21, 915-923; A. Zieris, S. Prokoph, K. R. Levental, P. B. Welzel, M. Grimmer, U. Freudenberg, C. Werner, Biomaterials 2010, 31, 7985-7994; U. Freudenberg, J.-U. Sommer, K. R. Levental, P. B. Welzel, A. Zieris, K. Chwalek, K. Schneider, S. Prokoph, M. Prewitz, R. Dockhorn, C. Werner, Advanced Functional Materials 2012, 22, 1391-1398; U. Freudenberg, A. Hermann, P. B. Welzel, K. Stirl, S. C. Schwarz, M. Grimmer, A. Zieris, W. Panyanuwat, S. Zschoche, D. Meinhold, Biomaterials 2009, 30, 5049-5060; M. V. Tsurkan, K. R. Levental, U. Freudenberg, C. Werner, Chemical Communications 2010, 46, 1141; K. Chwalek, K. R. Levental, M. V. Tsurkan, A. Zieris, U. Freudenberg, C. Werner, Biomaterials 2011, 32, 9649-9657; M. V. Tsurkan, K. Chwalek, K. R. Levental, U. Freudenberg, C. Werner, Macromol Rapid Commun 2010, 31, 1529-1533), in welchem Netzwerk-Eigenschaften graduell variiert werden können, während die Gehalte an Heparin konstant bleiben. Wie sich zeigte, korrelieren Maschenweite, Quellung und Elastizitätsmodule gut mit dem Grad der Gel-Komponenten-Vernetzung. Darüber hinaus erlaubte die sekundäre Umwandlung von Heparin innerhalb der biohybriden Gele die kovalente Anbindung von Zelladhäsion-vermittelnden RGD-Peptiden. Die biohybriden Gele wurden angewendet, um die Auswirkungen von mechanischen und biomolekularen Signalen auf primäre Nervenzellen und neuronale Stammzellen zu demonstrieren. Die Ergebnisse zeigen das zelltypspezifische Zusammenspiel von synergistischer Signalgebung und dem Potenzial der Biohybridmaterialien, die Zellschicksalsentscheidung selektiv zu stimulieren. In jüngster Zeit wurden in eigenen Arbeiten der Erfinder proteasesensitive und -insensitive Spaltstellen für die weitreichende Kontrolle über die Abbauraten von Stern-PEG-Heparin-Hydrogelnetzwerken mit orthogonal modulierter Elastizität, RGD-Peptidpräsentation und VEGF-Abgabe kombiniert. Die enzymatische Spaltung wurde massiv beschleunigt, wenn die Zugänglichkeit der Gele für Proteasen durch nichtenzymatische Spaltungen von Esterbindungen erhöht wurde. Die Auswirkungen der Abbauanfälligkeit des Gels wurden für das dreidimensionale Wachstum von humanen Endothelzellen untersucht. Gele mit beschleunigtem Abbau und einer VEGF-Freisetzung führten zu einer deutlichen Verstärkung des Eindringens von Endothelzellen *in vitro* wie auch der Blutgefäß-Dichte im Hühner-Chorioallantoismembran-Test (HET-CAM) *in vivo.* Somit kann die Kombination von proteasesensitiven und -insensitiven Spaltstellen den Abbau von bioresponsiven Gel-Materialien in einer Weise verstärken, dass die Morphogenese der Endothelzellen gesteigert wird.

Künstliche Proteinhydrogele, die durch die Interaktion von Leucin-Zipper-Domänen aufgebaut sind, haben die Fähigkeit zu einer durch die Proteinsequenzen bestimmten Selbstorganisation. **Tirrell et al.** (W. Shen, K. Zhang, J. A. Kornfield, D. A. Tirrell, Nature materials 2006, 5, 153-15) haben ein durch die Doppelwendeldomänen zusammengesetztes Hydrogel entwickelt. Untersuchungen der strukturellen und dynamischen Eigenschaften der AC10A-Hydrogele in geschlossenen Systemen ergaben, dass diese Multidomain-Proteinketten eine starke Tendenz aufweisen, intramolekulare Schlaufen zu bilden. Dies führt zu einer schnellen Gel-Erosion. Daher wurde das System verbessert, wobei belegt werden konnte, dass die Erosionsgeschwindigkeit des Proteinhydrogels durch Nutzbarmachung einer selektiven molekularen Erkennung, durch eine bestimmte Aggregationszahl und durch eine Orientierungs-Diskriminierung von Doppelwendelproteindomänen abgestimmt werden kann. Die Experimente haben gezeigt, dass die Wechselwirkung zwischen Molekülen während der Selbstorganisation und Gelierung nicht in so einfacher Weise funktioniert wie ein "Schlüssel-Schloss"-Prozess. Stattdessen bestimmt die Dynamik und Thermodynamik des gesamten Systems die physikalischen und biochemischen Eigenschaften der resultierenden Polymer-Matrices. Da solche physikalischen und chemischen Parameter in dem Polymer-System nicht einfach untersucht und vorhergesagt werden können, wurde durch eigene Arbeiten der Erfinder der vorliegenden Erfindung eine Screening-Methode angewendet, um durch die Synthese vieler verschiedener Peptide und die Untersuchung ihrer Struktur-Funktions-Beziehung ein optimales selbstorganisierendes System von Matrices zu finden.

Die Erkennung zwischen den Basenpaaren aus zwei komplementären DNA-Sequenzen ist wahrscheinlich die am besten charakterisierte und am breitesten angewendete Wechselwirkung zwischen Biomolekülen. Diese Basenpaarerkennung ist nicht nur das Thema einer Vielzahl von genetischen und biochemischen Forschungen, sondern auch ein zunehmend nützliches Werkzeug in den Materialwissenschaften. Zum Beispiel wurde die für die Konstruktion von Nanostrukturen beliebiger Form und Topologie viel versprechende DNA-Origami-Technik entwickelt. Durch DNAselbstorganisierende und/oder enzymkatalysierte DNA-Ligation wurden unlängst DNA-basierte Hydrogelsysteme entwickelt. **Luo et al.** (S. H. Um, J. B. Lee, N. Park, S. Y. Kwon, C. C. Umbach, D. Luo, Nature materials 2006, 5, 797-801) haben über die vollständige Konstruktion eines Hydrogels aus verzweigter DNA berichtet. Da die DNA eine wesentliche Komponente in der Biologie ist, sind diese DNA-Hydrogele biokompatibel, biologisch abbaubar, kostengünstig herstellbar und können in einfacher Weise in die gewünschten Formen und Größen gebracht werden. Gelierungs-Prozesse der DNA können unter physiologischen Bedingungen erreicht werden. Die Umhüllungen von Proteinen und Zellen können *in situ* durchgeführt werden. Ferner kann die Feinabstimmung dieser Hydrogele durch Anpassen der anfänglichen Konzentrationen und Arten von verzweigten DNA-Monomeren erreicht werden. Das wichtigste Ergebnis bestand darin, dass die sich daraus ergebenden Polymermatrices hoch definierte Strukturen im Nanometerbereich gezeigt haben, in guter Übereinstimmung mit der Vorhersage bezüglich der DNA-Doppelhelix-Struktur.

Nachteiligerweise hat sich das Hydrogelsystem, das auf Peptid-Stern-PEG-Konjugaten und LMWH-Stern-PEG-Konjugaten basiert, als sehr weich gezeigt, womit es für viele Konstruktionsprozesse ungeeignet ist. Die Peptidsequenzen des AT-III-Peptids und des HIP-Peptids stammen jeweils aus den Heparin-bindenden Proteinen Antithrombin III (ATIII) und HIP (HIP = Heparin/heparan sulfate interacting protein), welche jeweils selbst biologische Aktivitäten aufweisen. In ähnlicher Weise haben der dimere Wachstumsfaktor beziehungsweise das VEGF-Gel auch das potenzielle Risiko, eine unerwünschte Reaktion von Zellen oder vom Wirt hervorzurufen. So können das Gel aus den dimeren Wachstumsfaktoren und die dimeren Wachstumsfaktoren selbst stark toxisch sein, weil Wachstumsfaktoren nur in sehr niedrigen Konzentrationen im Körper vorkommen und auch in sehr niedrigen Konzentrationen wirken. Eine Überdosis ist sehr gefährlich und kann von Krebs bis zum sofortigen Tod führen. Verschiedene proteinbasierte Hydrogelsysteme bergen auch das potenzielle Risiko, eine Immunantwort hervorzurufen, da die künstlichen Multi-Domain-Proteine durch das Wirtsimmunsystem als fremde Antigene erkannt werden können. Das DNA-Hydrogel kann mit vertretbaren Kosten für eine Labor-Anwendung hergestellt werden, obwohl die Synthese im großen Maßstab sehr teuer werden könnte. Obwohl es chemisch möglich ist, andere bioaktive funktionelle Gruppen und/oder chemisch/physikalisch reaktive Gruppen in das DNA-Hydrogel zu übernehmen, würde dies jedoch die Herstellungskosten beträchtlich erhöhen. Die meisten chemischen Vernetzungsreaktionen für die Polymerisation würden auch zu einer Modifikation der Zelloberflächenmoleküle führen und für Zellen toxisch sein. Die Thiol-En- oder Thiol-Maleimid-Additionsreaktionen sind dadurch relativ mild, so dass das Alken und das Maleimid mit freien Thiolgruppen von Zelloberflächenproteinen reagieren können, während die Thiolgruppe in dem Polymer eine Disulfidbindungsaustauschreaktion mit dem Disulfid-Bindungen enthaltenden Zelloberflächenprotein haben wird. Kupferfreie Klick-Chemie stellt die am besten geeignete Strategie für die chemische *in situ* Gelbildung dar. Jedoch ist die Cyklooctin-Struktur sehr lipophil und könnte einen hydrophoben Cluster in einer Polymermatrix bilden. Darüber hinaus sind der Metabolismus und die Toxizität der resultierenden Triazolstruktur unbekannt und müssen in aufwendigen biologischen und klinischen Tests nachgewiesen werden.

Die WO2008/124165 A2 beschreibt ein selbstorgansierendes Nanopartikel-Arzneimittelabgabesystem für die Abgabe von verschiedenen bioaktiven Wirkstoffen, einschließlich Peptiden, Proteinen, Nukleinsäuren oder synthetischen chemischen Medikamenten. Eine Dipeptid-Sequenz (KA) wird dafür verwendet, DNA und RNA auf kleine Partikel zu binden.

Die Druckschriften Yamaguchi, N. et al.: Rheological Characterization of Polysaccharide-Poly(ethylene glycol) Star Copolymer Hydrogels. Biomacromolecules, 2005, 6,1931-1940**;** und Yamaguchi, N., Kiick, K. L.: Polysaccharide-Poly(ethylene glycol) Star Copolymer as a Scaffold for the Production of Bioactive Hydrogels. Biomacromolecules, 2005, 6, 1921-1930**;** lehren jeweils ein nicht-kovalentes Hydrogel auf der Basis einer Peptid-Oligosaccharid-Interaktion, wobei jeweils ein Peptid mit einem sich wiederholenden Dipeptid-Motiv (KA)₄ verwendet wird.

Aus der US 6 958 212 B1 sind polymere Biomaterialien, zum Beispiel Hydrogele, bekannt, die durch nukleophile Additionsreaktionen an konjugierte ungesättigte Gruppen gebildet werden und pharmazeutisch wirksame Einheiten enthalten. Die Komponenten des Biomaterials können aus einer Gruppe ausgewählt werden, die aus Oligomeren, Polymeren, biosynthetischen Proteinen oder Peptiden, natürlich vorkommenden Proteinen oder Peptiden, modifizierten natürlich vorkommenden Peptiden oder Proteinen und Polysacchariden besteht.

In der Druckschrift Alison B. Pratt et al.: Synthetic extracellular matrices for in situ tissiu engineering. Biotechnology and Bioengineering, 2004, 86, 27-36**;** ist die Formung eines Hydrogels als synthetische extrazellulare Matrix beschrieben. wobei das Hydrogelnetzwerk aus einem Heparin-bindenden Peptid und PEG gebildet werden kann.

Ähnliche Hydrogelsysteme sind in der Druckschrift Kyung Jae Jeong et al.: Interplay between Covalent and Physical Interactions within Enviroment Sensitive Hydrogels. Biomacromolecules, 2009, 10, 1090-1099**;** der Druckschrift Seal B. L. et al. Physical matrices stabilized by enzymatically sensitive covalent crosslinks. Acta Biomaterialia, 2006, 2, 241-251**;** der Druckschrift Brandon L. Seal et al.: Physical Polymer Matrices Based on Affinity Interactions between Peptides and Polysaccharides. Biomacromolecules, 2003, 4, 1572-1582**;** sowie der Druckschrift Brandon L. Seal et al.: Viscoelastic Behavior of Enviromentally Sensitive Biomimetic Polymer Matrices, Macromolecules, 2006, 39, 2268-2274 offenbart. Als Heparin-bindendes Peptid wird dabei jeweils Antithrombin III (ATIII) eingesetzt. Weitere Druckschriften, wie die Druckschrift M. V Tsurkan et al.: Enzymatically degradable heparin-polyethylene glycol gels with controlled mechanical properties, Chemical Communications, 2010, 46, 1141-1143**;** die Druckschrift M. V. Tsurkan et al.: Modular StarPEG-Heparin Gels with Bifunctional Peptide Linkers. Macromol Rapid Commun, 2010, 31, 1529-1533**;** die Druckschrift U. Freudenberg et al.: A star-PEG-heparin hydrogel platform to aid cell replacement therapies for neurodegenertative diseases. Biomaterials, 2009, 30, 5049-5060**;** die Druckschrift T. Nie et al. Production of heparin-containing hydrogels for modulating cell responses. Acta Biomaterialia, 2009, 5, 865-875**;** sowie die Druckschrift Benoit et al.: The effect of heparin-functionalized PEG hydrogels on three-dimensional human mesenchymal stem cell osteogenic differentiation. Biomaterials, 2007, 28, 66-77**;** befassen sich mit kovalenten Heparin-anhaftenden Hydrogelen, die enzymatisch spaltbare Linker und Zellanhaftungssequenzen aufweisen.

Die WO 94/04176 A1 beschreibt Peptide zur Neutralisierung/Bindung von Heparin zur Verbesserung der Blutkoagulation. Dabei werden Sequenzen mit Glycin eingesetzt.

Die Aufgabe der Erfindung besteht darin, synthetische Systeme von Polymermatrices mittels eines rationalen Design-Konzeptes bereitzustellen. Mit diesem System soll eine Verbesserung der Eigenschaften für biologische und klinische Anwendungen erreicht werden.

Die Lösung der Aufgabe der Erfindung besteht in einer nichtkovalenten selbstorganisierenden Hydrogelmatrix für biotechnologische Anwendungen, enthaltend ein kovalentes Polymer-Peptid-Konjugat, wobei das kovalente Polymer-Peptid-Konjugat Konjugate von zwei oder mehr Peptiden umfasst, die an eine Polymerkette gekoppelt sind. Die Peptid-Sequenz enthält ein sich wiederholendes Dipeptid-Motiv (BA)ₙ, worin B eine Aminosäure mit positiv geladener Seitenkette, A Alanin und n eine Zahl von 5 bis 20, die die Anzahl der einzelnen sich wiederholenden Dipeptid-Module (BA) innerhalb des Dipeptid-Motivs (BA)ₙ angibt, ist. Die Aminosäure B ist dabei vorzugsweise Arginin, mit dem Ein-Buchstaben-Code R, oder Lysin, gekennzeichnet mit dem Ein-Buchstaben-Code K. Das erfindungsgemäße Hydrogel eignet sich zur Bildung einer nichtkovalenten Hydrogelmatrix, die aufgrund der Bildung des kovalenten Polymer-Peptid-Konjugates selbstorganisierende Eigenschaften aufweist.

Mit der Erfindung wird somit ein sich *in situ* bildendes Hydrogelsystem bereitgestellt. Die Polymer-Matrices können durch einfaches Mischen von zwei Komponenten, die vollständig kompatibel mit Zell-Einbettungsexperimenten sind, gebildet werden. Es wurde darüber hinaus eine Reihe von Peptid-Polymer-Konjugaten untersucht, um deren Fähigkeit zu testen, sich mit einem Oligosaccharid zu verbinden, um ein Hydrogel zu bilden. Diese Vorgehensweise hat nicht nur zu einer Reihe von Gel-Systemen mit unterschiedlichen physikalischen, chemischen und biologischen Eigenschaften geführt, sondern auch zu einem Einblick in die Struktur-Funktions-Beziehung. Daher wurden chemische, physikalische, biochemische und biologische Tests zu den resultierenden Hydrogelen durchgeführt. Da die Peptidsequenzen auf dem einfachen (BA)ₙ-Motiv basieren, haben Untersuchungen zur Struktur-Funktionsbeziehung gezeigt, dass sehr einfache Änderungen in den Sequenzen zu einer Vielzahl von Gel-Eigenschaftsänderungen führen können.

Gemäß einer Ausführungsform der Erfindung wird die Polymerkette aus einem linearen oder mehrarmigen Polyethylenglykol (PEG) gebildet. Besonders bevorzugt ist dabei eine Ausführungsform, bei der die Polymerkette aus einem vierarmigen Polyethlenglykol (Stern-PEG) gebildet wird. Als Aminosäure B ist vorzugsweise Arginin oder Lysin vorgesehen. Außer L- und D-Aminosäuren von Arginin und Lysin der natürlichen Aminosäuren sind grundsätzlich aber quasi alle nicht-natürlichen Aminosäuren, welche positiv geladen (basisch) sind, geeignet.

Entsprechend der Erfindung umfasst die Hydrogelmatrix ferner ein hoch negativ geladenes Oligosaccharid. Gemäß der Erfindung besteht ein Oligosaccharid/Peptid/Polymer-System, bei dem das Peptid an das Polymer chemisch konjugiert ist und die Gelbildung durch Mischen des Peptid-Polymer-Konjugates und des Oligosaccharides erfolgt. Die nichtkovalente makromolekulare Selbstorganisation wird auch durch die Wechselwirkung zwischen dem Peptid und dem Oligosaccharid induziert. Die Wahl des Polymers und Oligosaccharids kann zu verschiedenen Gel-Eigenschaften, einschließlich des Fließverhaltens, des Gelierungszustandes und der Gelierungsgeschwindigkeit sowie einstellbarer Affinität von mit Oligosacchariden wechselwirkenden bioaktiven Proteinen, zum Beispiel Wachstumsfaktoren oder Morphogen, führen. Allerdings wird die größte Vielfalt an Gel-Eigenschaften überraschenderweise durch Änderungen eines sehr einfachen und sich wiederholenden Peptidsequenzmotivs erreicht, womit nach der Konzeption der Erfindung eine entsprechende Hydrogelmatrix prinzipiell auch ohne Oligosaccharid möglich ist. Auf diese Weise kann die flexible Gestaltung der Peptidsequenz zu einer breiten Vielfalt von Gel-Eigenschaften führen, nicht nur zu den oben genannten rheologischen Eigenschaften, dem Gelierungszustand, der Gelierungsgeschwindigkeit und Proteinbindungseigenschaften, sondern auch zu Eigenschaften, wie zum Beispiel der biologischen Abbaubarkeit aufgrund der proteolytischen Hydrolyse oder anderer enzymatischer Aktivität sowie der Empfindlichkeit gegenüber Lichtbestrahlung.

Das hoch negativ geladene Oligosaccharid ist gemäß einer vorteilhaften Ausführungsform ein sulfatiertes oder phosphoryliertes Oligosaccharid, vorzugsweise aus einer Gruppe von Oligosacchariden ausgewählt, die Heparin, Dextransulfat, α-Cyclodextrinsulfat, β-Cyclodextrinsulfat, γ-Cyclodextrinsulfat, α-Cyclodextrinphosphat, β-Cyclodextrinphosphat und γ-Cyclodextrinphosphat umfasst. In einer besonders bevorzugten Ausführungsform für ein Oligosaccharid/Peptid/Polymer-System umfasst die Hydrogelmatrix als Oligosaccharid Heparin, das aus Schleimhautgewebe von Schweine-Darm oder Rinderlunge stammt. Heparin ist dabei vorzugsweise von pharmazeutischer Qualität. In einer alternativen Ausführungsform umfasst die Hydrogelmatrix als Oligosaccarid Dextransulfat, das vorzugsweise ein Molekulargewicht im Bereich von 4 kDa bis 600 kDa aufweist. Bevorzugt wird der Einsatz von Dextransulfat pharmazeutischer Qualität. Enthält die Hydrogelmatrix Cyclodextrinsulfat, ist dieses vorzugsweise α-Cyclodextrinsulfat, β-Cyclodextrinsulfat oder γ-Cyclodextrinsulfat von pharmazeutischer Qualität, wobei der Sulfatierungsgrad von drei Sulfaten pro Molekül bis zur vollständigen Sulfatierung betragen kann. Enthält die Hydrogelmatrix Cyclodextrinphosphat, ist dieses vorzugsweise α-Cyclodextrinphosphat, β-Cyclodextrinphosphat oder γ-Cyclodextrinphosphat von pharmazeutischer Qualität, wobei der Phosphorylierungsgrad von drei Phosphatgruppen pro Molekül bis zur vollen Phosphorylierung betragen kann.

Entsprechend einer weiteren Ausgestaltung der Erfindung umfasst die Hydrogelmatrix ferner eine unter Licht spaltbare chemische Gruppe zwischen der Polymerkette und der Peptid-Sequenz, die das sich wiederholende Dipeptid-Motiv (BA)ₙ enthält. Die Hydrogelmatrix kann ferner auch einen pH-empfindlichen chemischen Linker zwischen der Polymerkette und der Peptid-Sequenz umfassen, die das sich wiederholende Dipeptid-Motiv (BA)ₙ enthält. Gemäß einer anderen Ausführungsform der Erfindung umfasst die Hydrogelmatrix ferner einen enzymatisch spaltbaren Linker zwischen der Polymerkette, vorzugsweise ein PEG-Molekül, und der Peptid-Sequenz, die das sich wiederholende Dipeptid-Motiv (BA)ₙ enthält. Als enzymatisch spaltbaren Linker umfasst die Hydrogelmatrix dabei vorzugsweise eine Peptidsequenz, die ein proteolytisch aktives Substrat ist. Alternativ kann die Hydrogelmatrix als enzymatisch spaltbaren Linker ferner eine Oligonukleotid-Sequenz umfassen, die ein Nuklease-aktives Substrat ist.

Die Modifizierung des Peptids kann zum weiteren Ausbau der Hydrogelfunktion durch die Einführung verschiedener Markierungen, zum Beispiel Fluoreszenz-Markierung für die Überwachung der Matrices *in vitro* und *in vivo,* und zum weiteren Ausbau der Wirkstoff-Konjugation für eine Wirkstoff-Freisetzung führen. Ferner, da die Gelbildung durch zwei chemisch definierte Komponenten induziert wird, kann das Matrix-System Schicht für Schicht ausgebildet werden, um ein Peptid-Polymer-Konjugat und/oder ein Oligosaccharid einer bestimmten Funktion an einer bestimmten Schicht mit hoher Präzision zu platzieren. Dieses Schicht-für-Schicht-Verfahren ermöglicht in Kombination mit der oben erwähnten lichtempfindlichen Ausführung der Hydrogelmatrix darüber hinaus den Entwurf und die Konstruktion von anspruchsvollen bioaktiven und biokompatiblen Nanostrukturen und Nanoeinheiten.

Alle Komponenten im erfindungsgemäßen Hydrogelsystem können bei vergleichsweise geringen Kosten erhalten oder hergestellt werden. Heparin, Dextransulfat und Cyclodextrinsulfat sind ebenso wie Maleimid-funktionalisiertes PEG-Polymer zu einem relativ niedrigen Preis bei kommerziellen Anbietern erhältlich. Peptide können mit einem Fest-Phasen-Peptid-Synthesizer im Gramm-Maßstab synthetisiert werden. Somit kann auch das resultierende Hydrogel im Gramm-Maßstab im Labor mit relativ niedrigen Kosten hergestellt werden. Vorteilhafterweise weist die Hydrogelmatrix nach einer der oben beschriebenen Ausführungsformen ein Elastizitätsmodul von mindestens 10 Pa auf.

Das selbstorganisierende System kann auch in einem Mikrofluid-System verwendet werden, um Hydrogelmatrixkugeln sowie in die Hydrogelmatrixkugeln einzubettende Zellen herzustellen. Ein weiterer Aspekt der Erfindung betrifft somit Hydrogelmatrixkugeln mit einem erfindungsgemäßen, eine selbstorganisierende Matrix bildenden Hydrogel. In eine mit dem erfindungsgemäßen Hydrogel gebildeten selbstorganisierenden Hydrogelmatrix oder in erfindungsgemäße Hydrogelmatrixkugeln können, wie bereits erwähnt, über ein entsprechendes Verfahren Zellen eingebettet werden, so dass ein entsprechender Verbund entsteht. Dabei werden die Zellen vorzugsweise aus einer Gruppe ausgewählt, die Säugetierzellen, Insektenzellen, Bakterien-Zellen und Hefezellen umfasst. Wenn die Zellen Säugetierzellen sind, können daraus vorteilhaft verschiedene Krebszelllinien, Fibroblastenzellen, pluripotente Stammzellen, induzierte pluripotente Stammzellen, humane T-Zellen oder menschliche B-Zellen ausgewählt werden. In eine erfindungsgemäße Hydrogelmatrix oder entsprechende Hydrogelmatrixkugeln eingebettete Zellen können zur Herstellung von Proteinen verwendet werden, wobei das Protein vorzugsweise therapeutische monoklonale Antikörper umfasst.

Ein weiterer Aspekt der Erfindung betrifft Kapseln zur gezielten Freisetzung von therapeutischen Reagenzien, wobei über ein entsprechendes Verfahren therapeutische Reagenzien mit der oben beschriebenen Hydrogelmatrix oder den oben genannten Hydrogelmatrixkugeln eingekapselt werden. Die Gruppe der jeweils verwendeten therapeutischen Reagenzien umfasst dabei vorzugsweise Säugetierzellen, Insektenzellen, Bakterien, Hefezellen, Anti-Krebs-Verbindungen, Anti-Koagulationsverbindungen, entzündungshemmende Verbindungen, immunsuppressive Verbindungen, therapeutische Antikörper, diagnostische Reagenzien, Hormone, Wachstumsfaktoren, Zytokine, kleine Moleküle als Hemmer für Wachstumsfaktoren, kleine Moleküle als Hemmer für Zytokine, Aptamer-Hemmer für Wachstumsfaktoren und Aptamer-Hemmer für Zytokine.

Ein weiterer Aspekt der Erfindung betrifft eine Zusammensetzung aus einer nichtkovalenten selbstorganisierenden Hydrogelmatrix in einer der oben genannten Ausführungsformen und therapeutischen Chemikalien und Reagenzien, wobei im entstehenden therapeutischen Hydrogel ein Gradient der Chemikalien und Reagenzien erzeugt ist. Das heißt, es ist möglich, durch ein geeignetes Verfahren einen Gradienten von Chemikalien und Reagenzien in den erfindungsgemäßen therapeutischen Hydrogelmatrices beziehungsweise Hydrogelmatrixkugeln zu erzeugen. Zu den möglichen therapeutischen Chemikalien und Reagenzien, die Gradienten in der Hydrogelmatrix bilden, zählen bevorzugt Anti-Koagulationsverbindungen, entzündungshemmende Verbindungen, immunsuppressive Verbindungen, therapeutische Antikörper, diagnostische Reagenzien, Hormone, Wachstumsfaktoren, Zytokine, kleine Moleküle als Hemmer für Wachstumsfaktoren, kleine Moleküle als Hemmer für Zytokine, Aptamer-Hemmer für Wachstumsfaktoren sowie Aptamer-Hemmer für Zytokine.

Schließlich betrifft ein weiterer Aspekt der Erfindung ein Hybridsystem aus einer erfindungsgemäßen Hydrogelmatrix, die nicht in Kugelform vorliegt einerseits und Hydrogelmatrixkugeln andererseits. Dabei weisen die nichtkugelförmige Hydrogelmatrix und die Hydrogelmatrixkugeln jeweils eine unterschiedliche chemische Zusammensetzung auf, und eine Komponente des Hybridsystems ist durch Bestrahlung mit Licht, durch selektiven chemischen Abbau oder durch enzymatische Verdauung regelbar.

Weitere Einzelheiten, Merkmale und Vorteile der Erfindung ergeben sich aus der nachfolgenden Beschreibung von Ausführungsbeispielen mit Bezugnahme auf die zugehörigen Zeichnungen. Es zeigen:
- **Fig. 1:**: eine schematische Darstellung eines Auswahlverfahrens zum Nachweis der Bildung eines Hydrogels mit Heparin,
- **Fig. 2:**: die Ergebnisse der Umkehrphasen- Ultrahochdruckflüssigkeitschromatographie (UPLC) von gereinigten Peptiden,
- **Fig. 3:**: die Ergebnisse der Umkehrphasen- Hochdruckflüssigkeitschromatographie (UPLC)-Analyse von gereinigten Peptid-Vierarm-Polyethylenglykol-Konjugaten (Peptid-Stern-PEG),
- **Fig. 4a-f:**: eine Analyse einer Heparin-abhängigen Strukturänderung durch Zirkulardichroismus-Spektroskopie (CD),
- **Fig. 5:**: eine schematische Darstellung der Hochdurchsatzanalyse der mechanischen Eigenschaften der Hydrogele,
- **Fig. 6:**: die Stabilität eines auf Lysin und Alanin basierenden Hydrogels mit Vergleich von L- und D-Aminosäuren,
- **Fig. 7a-b:**: das Fließverhalten von Peptid-Stern-PEG-Konjugat und 14-kDa-Heparin jeweils einzeln,

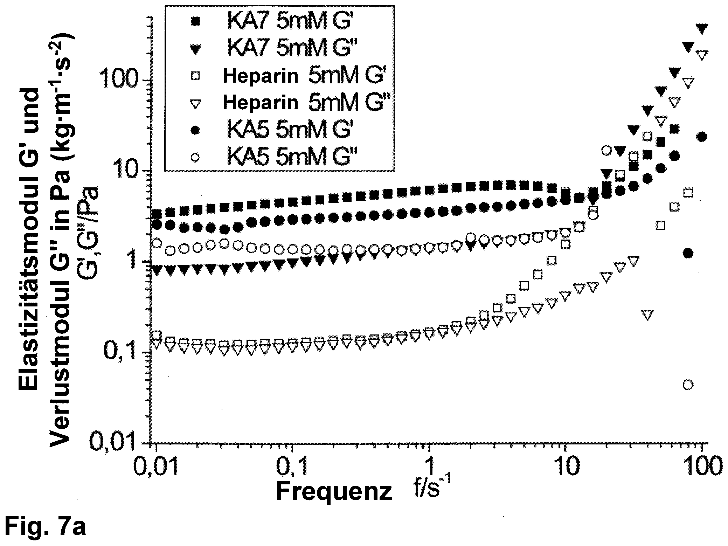

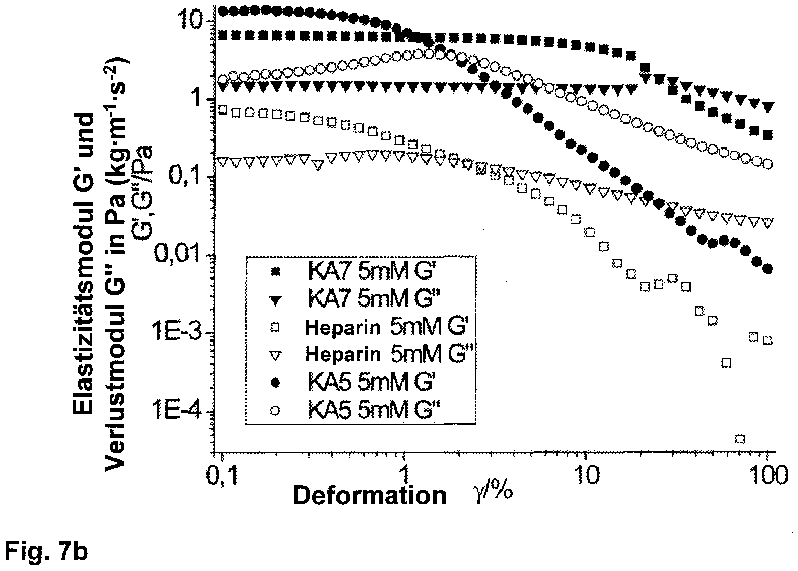

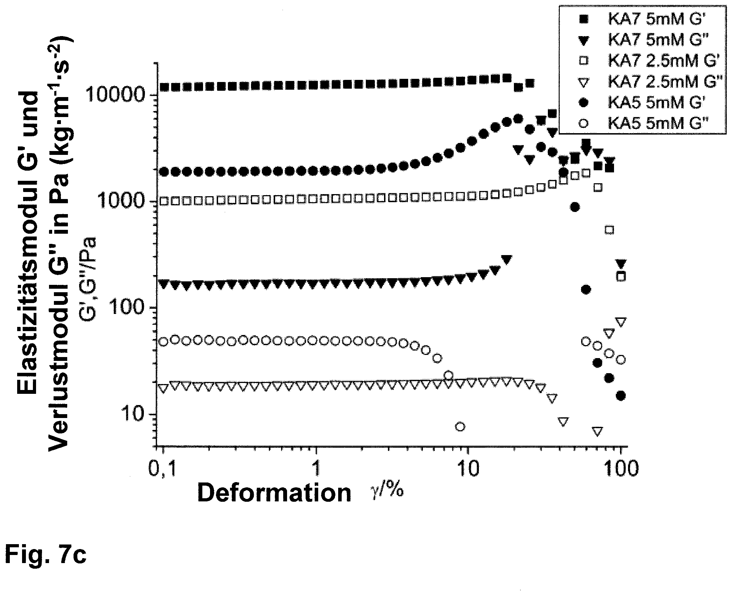

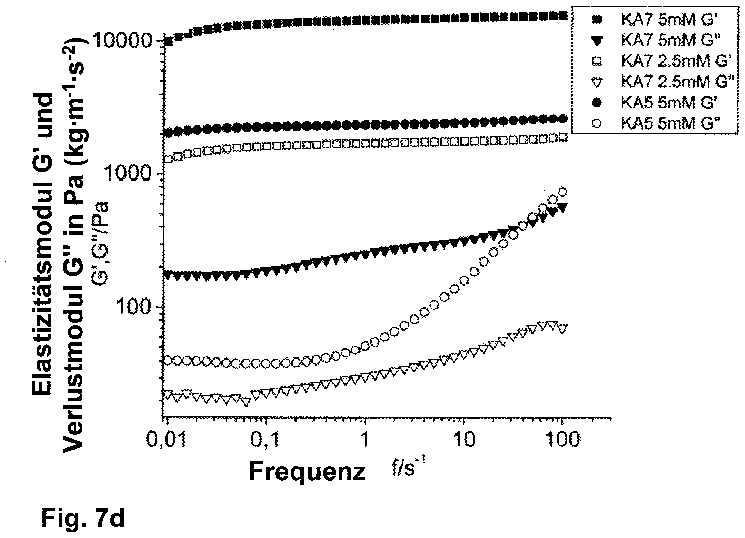

- **Fig. 7 c-d:**: das Fließverhalten von Peptid-Stern-PEG-Konjugat mit 14-kDa-Heparin,

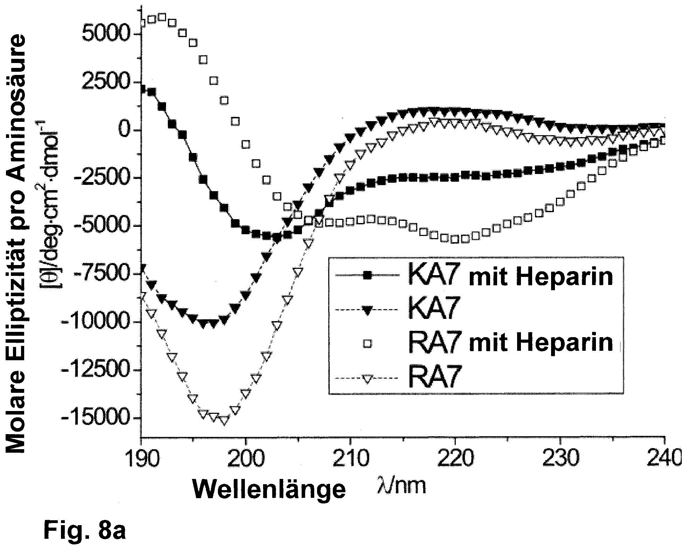

- **Fig. 8a:**: die Analyse einer Heparin-abhängigen Strukturänderung durch Zirkulardichroismus-Spektroskopie,

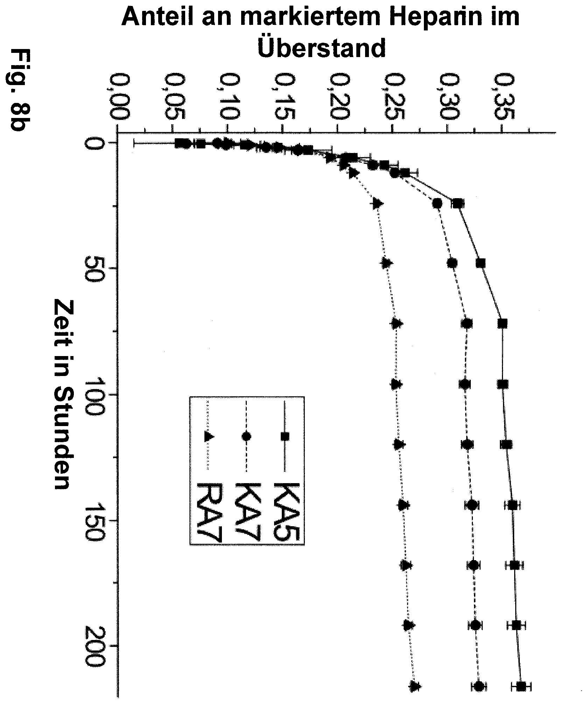

- **Fig. 8b:**: das Ergebnis der Überprüfung der Erosion des Hydrogels durch das Zusammenmischen von Peptid-Stern-PEG-Konjugat und mit TAMRA-markiertem 14-kDa-Heparin,

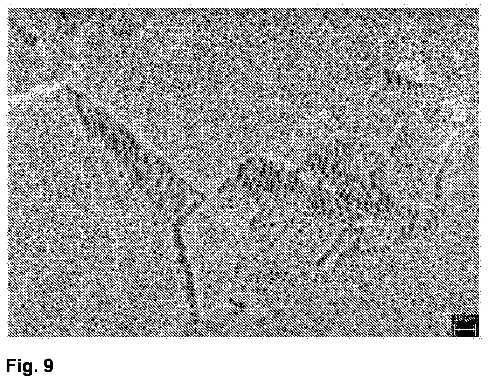

- **Fig. 9:**: eine Raster-Mikroskopie-Abbildung des KA7-Stern-PEG-Hydrogels mit Heparin,

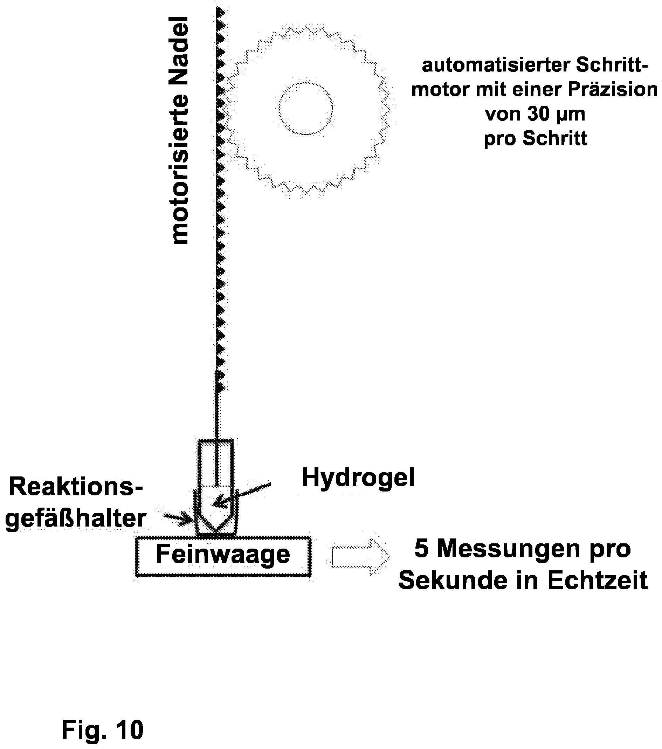

- **Fig. 10:**: eine Einrichtung zur Analyse der Gelierungszeit eines Hydrogels,

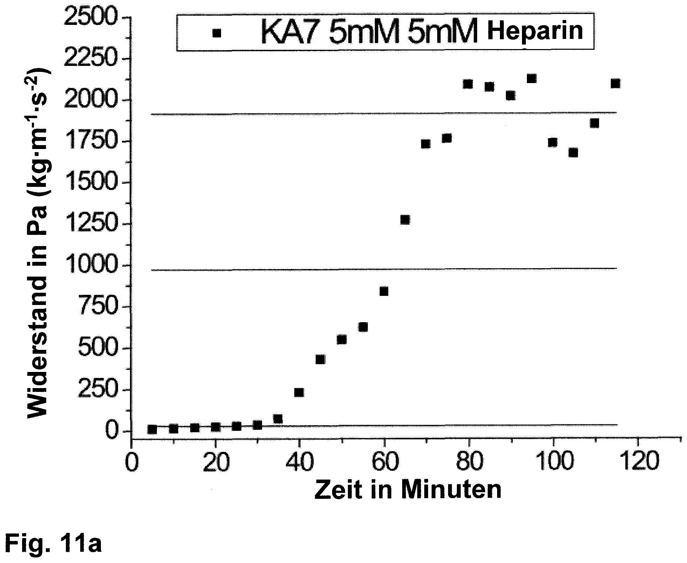

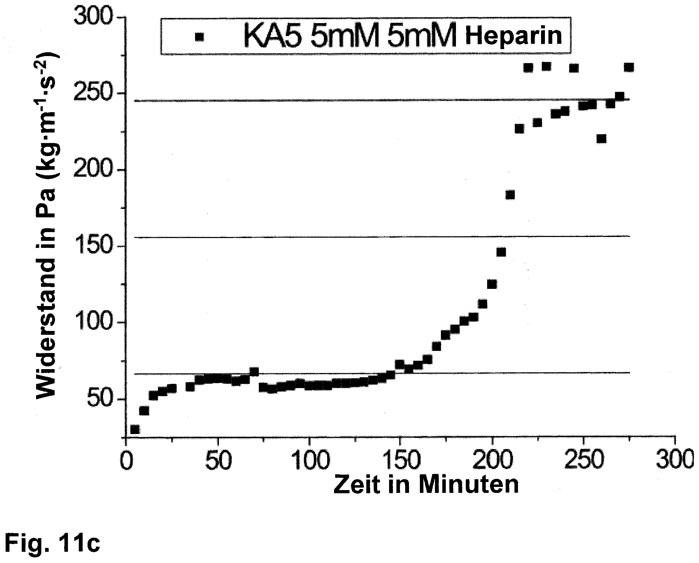

- **Fig. 11a-c:**: das Ergebnis der Analyse der Gelierungszeit eines Hydrogels,

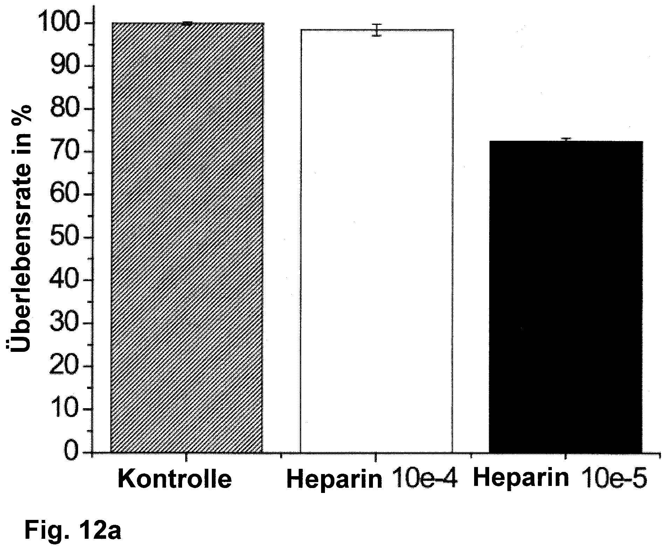

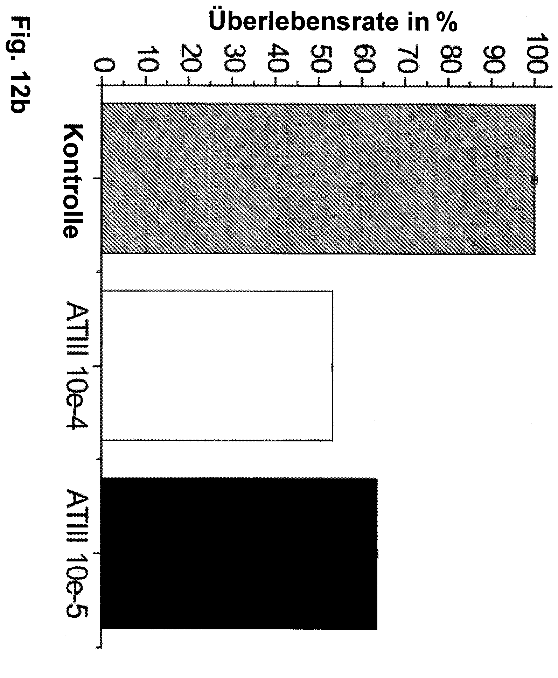

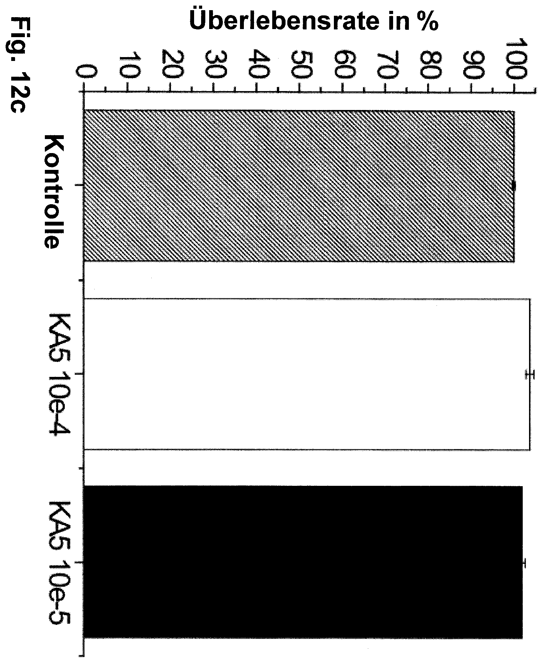

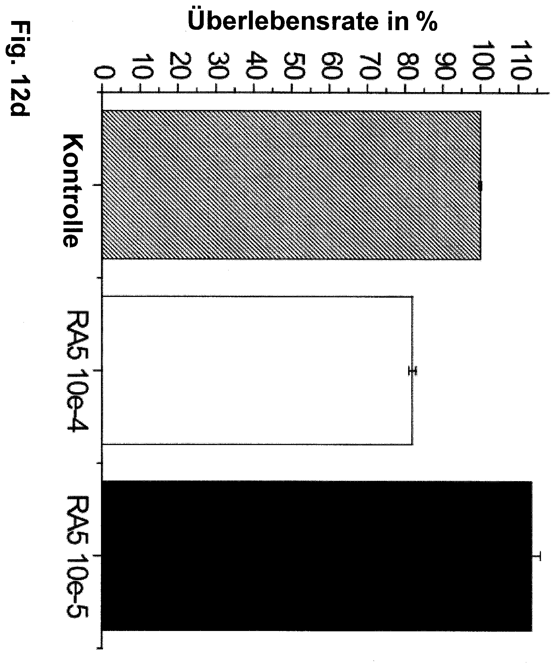

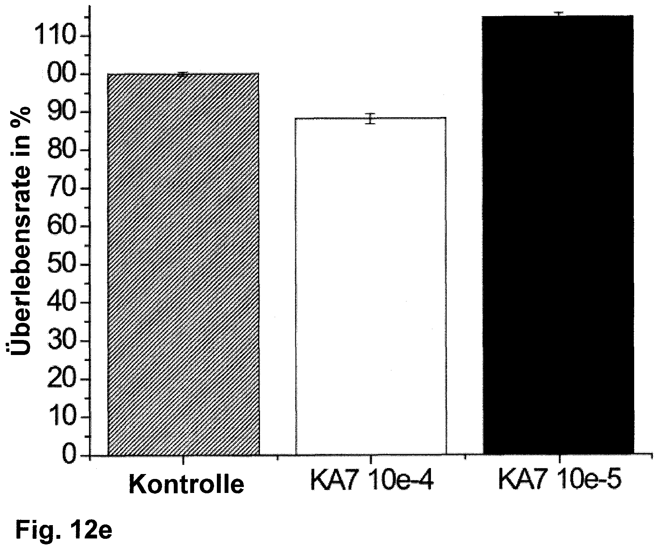

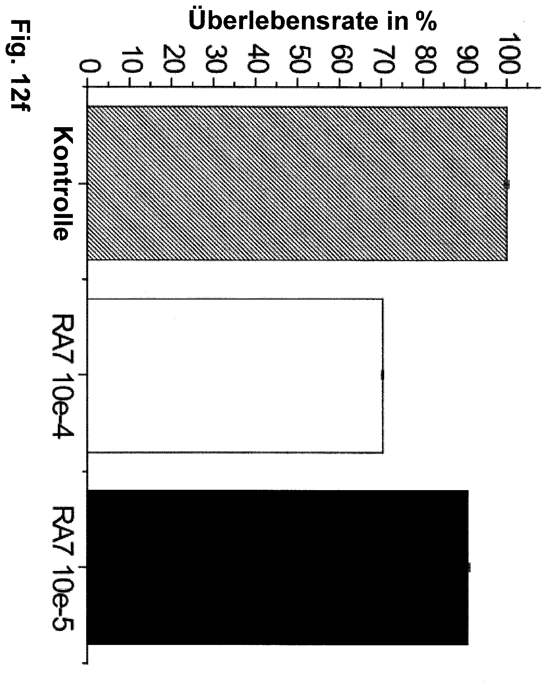

- **Fig. 12a-f:**: einen Toxizitätstest für verschiedene Peptid-Stern-PEG-Konjugate und 14-kDa-Heparin und

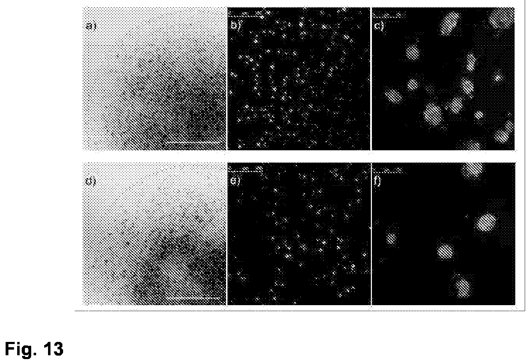

- **Fig. 13a-f:**: die Ergebnisse eines Lebensfähigkeitstests und die Struktur von eingebetteten menschlichen Fibroblasten (HDFn) aus der Haut von Neugeborenen in einem Hydrogel.

Ein einfaches, sich wiederholendes Peptidmotiv, was einfach modifiziert werden kann, um zu verschiedenen Bindungseigenschaften an bestimmten Biomolekülen zu führen, ist von großem Interesse in der Biochemie, Biotechnologie und in den Biomaterialwissenschaften. Beispielsweise kann ein solches System angewandt werden, um einstellbare selbstorganisierende nichtkovalente Matrixsysteme zu entwerfen. Heparin wurde als Ausgangsverbindung genutzt, um eine kovalente Hydrogelplattform zur Unterstützung von Zellersatztherapien zu synthetisieren. Im Folgenden wird eine Bibliothek von Peptiden vorgestellt, die jeweils an vierarmigem Polyethylenglykol (Stern-PEG) konjugiert sind, das in den Ausführungsbeispielen als Polymerkette dient. Die Bibliothek führt zur Ermittlung eines minimalen Heparin-bindenden Peptidmotivs (BA)ₙ, worin B ein Aminosäurerest, beispielsweise von Arginin oder Lysin ist, und worin A Alanin und n eine Zahl zwischen 4 bis 20 ist. Die Wiederholung dieses Motivs oder eine einzelne Aminosäuremutation führt zu einer Vielzahl von physikalischen und biochemischen Eigenschaften des resultierenden Heparin-abhängigen selbstorganisierenden Hydrogels.

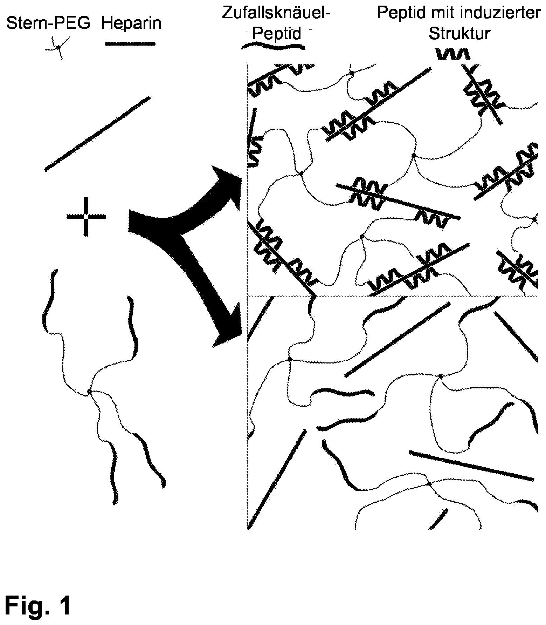

Die **Fig. 1** zeigt schematisch ein Auswahlverfahren zum Nachweis, dass die spezifischen Peptidmotive, die an ein vierarmiges Polyethylenglykol (Stern-PEG) gekoppelt sind, ein Hydrogel mit 14 kDa-Heparin bilden können. In der **Fig. 1** wird verdeutlicht, dass die Hydrogelbildung mit einer Heparin-induzierten Strukturänderung in dem (BA)ₙ-Peptidmotiv einhergeht.

Die **Tabelle 1** zeigt zunächst die Bibliothek synthetisierter Peptide. Gezeigt sind die Sequenzen, die Abkürzung und das Molekulargewicht der Peptide.

**Tabelle 1**

| Name | Sequenzkennzahl (SEQ ID NO) | Peptidsequenz | Molekulargewicht [10⁻³ kg/mol] |
|---|---|---|---|
| ATIII | 1 | CWGGKAFAKLAARL YRKA | 2010,44 |
| KA1 | 2 | CWGGKA | 620,72 |
| KA3 | 3 | CWGGKAKAKA | 1019,22 |
| KA5 | 4 | CWGGKAKAKAKAKA | 1417,72 |
| KA7 | 5 | CWGGKAKAKAKAKAKA KAKA | 1816,22 |
| dKdA7 | 6 | cwGGkakakakakakaka | 1816,22 |
| dKA7 | 7 | CWGGkAkAkAkAkAkA kA | 1816,22 |
| KdA7 | 8 | CWGGKaKaKaKaKaK aKa | 1816,22 |
| KA7-1a | 9 | CWGGKAKAKAKaKA KAKA | 1816,22 |
| KKA5 | 10 | CWGGKKAKKAKKAK KAKKA | 2058,57 |
| KG1 | 11 | CWGGKG | 606,69 |
| KG3 | 12 | CWGGKGKGKG | 977,13 |
| KG5 | 13 | CWGGKGKGKGKGK G | 1347,57 |
| KG7 | 14 | CWGGKGKGKGKGK GKGKG | 1718,01 |
| KKG5 | 15 | CWGKKGKKGKKGKK GKKG | 1988,42 |
| RA1 | 16 | CWGGRA | 648,74 |
| RA3 | 17 | CWGGRARARA | 1103,28 |
| RA5 | 18 | CWGGRARARARARA | 1557,82 |
| RA7 | 19 | CWGGRARARARARARA RARA | 2012,36 |
| RRA5 | 20 | CWGRRARRARRARR ARRA | 2338,77 |
| RG1 | 21 | CWGGRG | 634,71 |
| RG3 | 22 | CWGGRGRGRG | 1061,19 |
| RG5 | 23 | CWGGRGRGRGRGR G | 1487,67 |
| RG7 | 24 | CWGGRGRGRGRGR GRGRG | 1914,15 |
| RRG5 | 25 | CWGRRGRRGRRGR RGRRG | 2268,62 |

Im Folgenden sind die verwendeten Ein-Buchstaben-Codes den entsprechenden Aminosäuren sowie (in Klammern) deren Drei-Buchstaben-Codes gegenübergestellt:
▪ A ist die Abkürzung für Alanin (Ala),
▪ C für Cystein (Cys),
▪ F für Phenylalanin (Phe),
▪ G für Glycin (Gly),
▪ K für Lysin (Lys),
▪ L für Leucin (Leu),
▪ R für Arginin (Arg),
▪ W für Tryptophan (Trp) und
▪ Y für Tyrosin (Tyr).
L-Aminosäuren werden durch die Verwendung von Großbuchstaben, D-Aminosäuren durch die Verwendung von Kleinbuchstaben gekennzeichnet.

Alle in der **Tabelle 1** aufgeführten Peptide wurden unter Nutzung einer Standard-Fluorenylmethoxycarbonyl-Chemie (FMOC-Chemie) auf einer Festphase mit 2-(1H-Benzotriazol-1-yl)-1,1,3,3-Tetramethyluronoiumhexafluor-phosphat-Aktivierung (HBTU-Aktivierung) in einem automatischen Festphasen-Peptidsynthesizer (ResPep SL, Intavis, Köln, Deutschland) hergestellt. Für eine gute Peptidqualität wurde jede Aminosäure jeweils mit dem fünffachen Überschuss zweimal gekoppelt, wobei alle nicht reagierenden Aminogruppen mit Essigsäureanhydrid geschützt wurden. Für die Spaltung des Peptids vom Harz wurde das Harz eineinhalb Stunden mit einem Gemisch von Trifluoressigsäure(TFA)/Triisopropylsilan(TIS)/Wasser/Dithiothreitol(DTT) behandelt, wobei diese Komponenten im Verhältnis von 90 (v/v) : 2,5 (v/v) : 2,5 (v/v) : 2,5 (m/v) vorliegen.

Die Peptide wurden in Wasser gelöst, welches 2 mg/ml Tris(2-carboxyethyl)phosphin (TCEP) enthielt. Die Peptid-Reinigung wurde mittels Umkehrphasen-Hochdruckflüssigkeitschromatographie (UPLC) an einer präparativen HPLC-Anlage (ProStar™, Agilent Technologies, Santa Clara, USA) vorgenommen, die mit einer präparativen C18-Säule (AXIA™ 100A, Korngröße 10 µm, 250 x 30 mm, Phenomenex, Torrance, USA) ausgerüstet ist. Das Peptid wurde von der Säule unter Anwendung eines Gradienten von 5 % bis 100 % Lösungsmittel B bei 20 ml/min eluiert, wobei Lösungsmittel A 0,1 % Trifluoressigsäure (TFA) in Wasser und Lösungsmittel B 0,1 % TFA und 5 % Wasser in Acetonitril ist.

Die Reinheit wurde durch analytische Umkehrphasen-Ultrahochdruckflüssigkeitschromatographie (UPLC Aquity™ mit UV-Detektor, Waters, Milford Massachusetts, USA), ausgestattet mit einer analytischen C18-Säule (ACQUITY™ UPLC BEH C18, Korngröße 1,7 µm, 50 x 2,1 mm, Waters, Milford Massachusetts, USA), unter Anwendung eines isokratischen Gradienten und einer Elektrospray-Ionisations-Massenspektrometrie (ESI-MS) (ACQUITY™ TQ Detector, Waters, Milford Massachusetts, USA), bestätigt. Das Peptid wurde zu einem weißen Pulver gefriergetrocknet (CHRIST ALPHA™ 2-4 LD plus + vacuubrand RZ6) und bei 4 °C unter trockenen Bedingungen für nicht mehr als eine Woche vor der weiteren Verarbeitung gelagert.

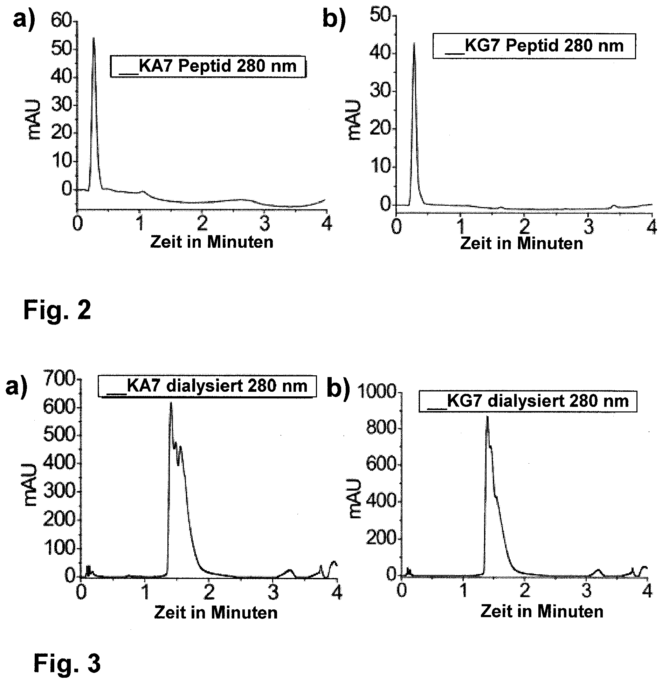

Die **Fig. 2** zeigt die Ergebnisse der Umkehrphasen-Ultrahochdruckflüssigkeitschromatographie (UPLC) von gereinigten Peptiden bei 280 nm unter Nutzung einer analytischen C18-Säule und eines isokratischen Gradienten. Die in der **Fig. 2** gezeigten Beispiel-Peptide aus der Bibliothek sind **a)** CWGGKAKAKAKAKAKAKA (KA7) und **b)** CWGGKGKGKGKGKGKGKG (KG7).

Die Synthese der für eine Hydrogel-Selbstorganisation genutzten Peptid-Stern-PEG-Konjugate wurde durch Michael-Additionsreaktionen zwischen Maleimidterminiertem vierarmigem PEG und Cystein-terminierten Peptiden aus der Bibliothek durchgeführt. Beide Komponenten wurden in physiologischer Phosphatpufferlösung (1XPBS) mit einem pH-Wert von pH 7,4 und in einem molaren Verhältnis von 1 : 4,5 (Stern-PEG : Peptid) mit einer Gesamtkonzentration von 80 mg/ml gelöst. Das Reaktionsgemisch wurde schnell verschlossen und bei 750 Umdrehungen pro Minute bei Raumtemperatur 18 Stunden lang gerührt (MR Hei-Standard, Heidolph, Schwabach, Deutschland). Die Rohprodukte wurden durch Umkehrphasen-Hochdruckflüssigkeitschromatographie (UPLC) (UPLC Aquity™ mit UV-Detektor, Waters, Milford Massachusetts, USA) unter Nutzung einer C18-Säule (ACQUITY™ UPLC BEH C18, Korngröße 1,7 µm, 50 x 2,1 mm, Waters, Milford Massachusetts, USA) und eines isokratischen Gradienten analysiert. Das Rohprodukt wurde zwei Tage lang gegen 10 Liter Wasser unter konstantem Wasseraustausch mit einer Dialysemembran mit einer Ausschlussgrenze (Cut-off) von 8 kDa dialysiert, um es von ungebundenen Peptiden und Salz zu befreien. Danach wurde das Produkt wieder in die UPLC eingespritzt, um die Reinheit im Vergleich zur Analyse vor der Dialyse zu überprüfen. Das dialysierte Produkt wurde in Wasser zu einem Feststoff gefriergetrocknet.

Die **Fig. 3** zeigt die Ergebnisse der Umkehrphasen-Hochdruckflüssigkeitschromatographie (UPLC)-Analyse von gereinigten Peptid-Vierarm-Polyethylenglykol-Konjugaten (Peptid-Stern-PEG) mittels einer UV-Detektion bei 280 nm. Die Ergebnisse sind in **Fig. 3** für die Beispielkonjugate aus der Bibliothek **a)** KA7-Stern-PEG und in **b)** KG-Stern-PEG gezeigt.

Im Folgenden ist die Herstellung der Hydrogelnetzwerke beschrieben. Dabei wurden 14-kDa-Heparin (25 mM, 2,5 mM) und Peptid-Stern-PEG-Konjugate (6,25 mM, 3,125 mM) in physiologischer Phosphatpufferlösung (1XPBS), Wasser oder Zellkulturmedium mit 2 % fetalem Rinderserum (FBS) gelöst.

Diese Lösungen wurden in einem Verhältnis von 1 : 4 Heparin : Peptid-Stern-PEG-Konjugat unter Erhalt von 0,5 mM oder 5 mM 14-kDa-Heparin und 2,5 mM oder 5 mM Peptid-Stern-PEG-Konjugat gelöst. Das Ligand-Molverhältnis war 2 : 1, 1 : 1 und 1 : 5 bezogen auf das Molverhältnis des 14-kDa-Heparin und der Peptid-Stern-PEG-Konjugate. Die Gemische wurden vor der Nutzung in einem Zeitraum von einer Stunde bis über Nacht bei Raumtemperatur oder 37 °C inkubiert. Die Gelierungungszeit reichte von sofort bis zu mehreren Stunden, was vom angewendeten Peptidmotiv abhängig war. Ein Hydrogel bildete sich, wenn es die Zugabe von physiologischer Phosphatpufferlösung (1XPBS) pH 7,4 zum Gemisch nach der Inkubation des Gemisches über die Nacht davor ohne Vermischung mit der hinzugegebenen Lösung überstand.

In **Tabelle 2** werden ausgewählte Peptide aus der Bibliothek aufgeführt, welche am besten die Strukturaktivitätsbeziehung der Hydrogelbildung mit Heparin reflektieren. ATIII ist ein aus der Literatur bekanntes Heparin-bindendes Peptid. Alle Peptide sind mit einem vierarmigen, Maleimid funktionalisierten 10-kDa-Polyethylenglykol (Stern-PEG) verbunden. Die Hydrogelbildung wurde in einem 50 µl-Gemisch getestet, welches 5 mM 14-kDa-Heparin und 5 mM (2,5 mM) Stern-PEG-Peptid-Konjugat in physiologischer Phosphatpufferlösung (1XPBS) pH 7,4. enthält. Die Deformations- und die Eindringgeschwindigkeit wurde durch Zentrifugieren des Hydrogels in einer 45°-Tischzentrifuge mit 275 µm Metallkügelchen auf der Oberfläche analysiert. Die Deformation der Oberfläche und das Eindringen der Kugeln wurden in Abhängigkeit von der aufgebrachten Kraft überwacht.

**Tabelle 2**

| Name | Peptidsequenz | Molekulargewicht [10⁻³ kg/mol] | Peptidmenge [10⁻³ mol/l] | Deformationsgeschwindigkeit [m/s²] | Eindringgeschwindigkeit [m/s²] |
|---|---|---|---|---|---|
| **mit Heparin gebildetes Gel** | | | | | |
| ATIII | CWGGKAFA KLAARLYR KA | 2010,44 | 5 | nicht bestimmt, Gel schrumpft | nicht bestimmt |
| KA5 | CWGGKAK AKAKAKA | 1417,72 | 5 | 11223 +/-4768 | 21209+/- 2188 |
| KA7 | CWGGKAK AKAKAKAK AKA | 1816,22 | 2,5 | 43998 +/-3139 | 72780+/- 6926 |
| KA7 | CWGGKAK AKAKAKAK AKA | 1816,22 | 5 | >148317 | 138919+/- 16275 |
| RA5 | CWGGRAR ARARARA | 1557,82 | 5 | <687 | <687 |
| RA7 | CWGGRAR ARARARAR ARA | 2012,36 | 5 | 1069 +/- 579 | 1952 +/-491 |

Die Heparin-bindende Domäne von Antithrombin III (ATIII) und Heparin mit niedrigem Molekulargewicht können ein weiches Hydrogel bilden, wenn beide an Stern-PEG konjugiert sind, wie bei N. Yamaguchi, B.-S. Chae, L. Zhang, KL Kiick, EM Furst, Biomacromolecules 2005, 6, 1931-1940**,** beschrieben. Um die chemische Komplexität zu verringern, wurden die Untersuchungen mit 14 kDa-Heparin durchgeführt. Es konnte festgestellt werden, dass in Gegenwart von ATIII-Peptid, welches an Stern-PEG konkugiert ist (ATIII-Stern-PEG), das resultierende Hydrogel sofort gebildet wird, aber dieses nicht das Gesamtvolumen bedeckt, wie aus **Tabelle 2** hervorgeht. Die Untersuchung von ATIII-Stern-PEG und Heparin zeigte, dass eine starke Wechselwirkung zwischen Heparin und Peptid nicht notwendigerweise zu einer optimalen Hydrogel-Netzwerkbildung führt. Daher wurde die Bibliothek von Peptid-Stern-PEG-Konjugaten erstellt, um Peptidsequenzen auf ihre Heparin-abhängigen selbstorganisierenden Eigenschaften zu untersuchen.

Die (BA)ₙ-Sequenz eröffnet die Möglichkeit, die Peptidlänge und damit die Eigenschaften leicht zu ändern, weshalb diese Sequenz als eine Basis ausgewählt wurde. Wie aus R. Tyler-Cross, RB Harris, M. Sobel, D. Marques, Protein Science 1994, 3, 620-627**,** bekannt ist, wechselt das ATIII-Peptid nach einer Heparin-Bindung aus einem Zufallsknäuel zu einer α-Helix, wobei auch für das (BA)ₙ erwartet wird, dass es bevorzugt eine α-Helixstruktur übernimmt. Es wurde überraschenderweise gefunden, dass das (BA)ₙ-Motiv eine minimale Sequenz-Anforderung für die Wechselwirkung mit Heparin darstellt.

Dem Ziel folgend, die größtmögliche Flexibilität hinsichtlich der Eigenschaften zu erreichen, wurden verschiedene Wiederholungen von (BA)ₙ synthetisiert, die in den oberen **Tabellen 1** und **2** aufgeführt sind. Einzelne Wiederholungen wurden als (negative) Gegenkontrolle verwendet, weil spiralförmige Formationen mindestens 5 Aminosäuren benötigen (α-Helix nach Pauling-Corey-Branson). Um außer Längen-Ladungs-Abhängigkeiten unter Berücksichtigung der Bindung an Heparin auch Ladungsdichte-Abhängigkeiten vergleichen zu können, wurden (BBA)₅ synthetisiert **(Tabelle 1).** (BBA)₅ weisen bei ähnlicher Länge eine höhere Ladungsdichte auf als (BA)₇. Wie bereits erwähnt, haben B und A die Tendenz zur Bildung von α-helikalen Strukturen, wie aus C. Nick Pace, J. Martin Scholtz, Biophysical Journal 1998, 75, 422-427**,** bekannt ist. Um immer ein Tandem von potentiell strukturbildenden und nicht-strukturbildenden Peptiden zu erhalten, hatte, wie auch **Tabelle 1** zeigt, jedes (BA)ₙ und (BBA)₅ einen (BG)ₙ- und einen (BBG)₅-Partner, wobei der Buchstabe G für Glycin steht. Glycin ist dafür bekannt, dass es jede Art von Strukturbildung stört. So hatte jedes der 20 intelligent gestalteten Mitglieder der Bibliothek eine Aufgabe zu erfüllen.

Zusätzlich zu dem Peptid-Motiv wurde ein Tryptophan, gekennzeichnet mit dem Ein-Buchstaben-Code W, für UV-Detektion und Reinigung und ein Cystein, gekennzeichnet mit dem Ein-Buchstaben-Code C, mit zwei Glycinen an das N-terminale Ende des Peptids gebunden. Durch die Anwendung der Michael-Additionschemie wurde das Cystein an ein Maleimid-funktionalisiertes 10-kDa-Stern-PEG gebunden. Synthese und Kopplung der Peptid-Stern-PEG-Konjugate sind hinsichtlich Reinheit, Schnelligkeit und der einfachen Handhabung optimiert worden, wie die **Fig. 3** zeigt. Dies ist die größte Bibliothek von Peptid-Polymer-Konjugaten, für die jeweils eine Oligosaccharidabhängige Hydrogelbildung analysiert wurde. Um die Bildung von Hydrogelen zu analysieren, wurden alle Peptid-Stern-PEG-Konjugate jeweils mit 14-kDa-Heparin in 50 µl physiologischem Phosphatpuffer (1XPBS) bis zu einer Endkonzentration von 5 mM gemischt. Nach Inkubation über Nacht wurde der physiologische Phosphatpuffer (1XPBS) hinzugefügt, um zu analysieren, welche Mischungen ein Hydrogel gebildet hatten. KA7-, KA5-, RA7- und RA5-Stern-PEG-Konjugate mit Heparin mischten sich nicht in 1XPBS, sondern bildeten ein stabiles klares Hydrogel, wie **Tabelle 2** zeigt. Das sind die kürzesten *de novo* hergestellten, in der Literatur bekannten Peptide, die ein Heparin-abhängiges Hydrogel bilden.

In **Tabelle 3** werden Peptide aus der Peptid-Bibliothek gezeigt, die kein Heparin-gebundenes Hydrogel bilden. Alle Peptide sind an ein 10-kDa-Maleimid-Stern-PEG gebunden. Die Gelbildung wurde in einem 50 µl-Gemisch getestet, das 5 mM 14 kDa-Heparin und 5 mM Peptid-Stern-PEG-Konjugat in 1XPBS bei einem pH-Wert von pH 7,4 enthält.

**Tabelle 3**

| Name | Peptid sequenz | Molekulargewicht [10⁻³ kg/mol] |
|---|---|---|
| **nicht mit Heparin gebildet** | | |
| KA1 | CWGGKA | 620,72 |
| KA3 | CWGGKAKAKA | 1019,22 |
| dKA7 | CWGGkAkAkAkAkAkAkA | 1816,22 |
| KdA7 | CWGGKaKaKaKaKaKaKa | 1816,22 |
| KA7-1a | CWGGKAKAKAKaKAKAKA | 1816,22 |
| KKA5 | CWGGKKAKKAKKAKKAKKA | 2058,57 |
| KG1 | CWGGKG | 606,69 |
| KG3 | CWGGKGKGKG | 977,13 |
| KG5 | CWGGKGKGKGKGKG | 1347,57 |
| KG7 | CWGGKGKGKGKGKGKGKG | 1718,01 |
| KKG5 | CWGGKKGKKGKKGKKGKKG | 1988,42 |
| RA1 | CWGGRA | 648,74 |
| RA3 | CWGGRARARA | 1103,28 |
| RRA5 | CWGGRRARRARRARRARRA | 2338,77 |
| RG1 | CWGGRG | 634,71 |
| RG3 | CWGGRGRGRG | 1061,19 |
| RG5 | CWGGRGRGRGRGRG | 1487,67 |
| RG7 | CWGGRGRGRGRGRGRGRG | 1914,15 |
| RRG5 | CWGGRRGRRGRRGRRGRRG | 2268,62 |

Es ist bemerkenswert, dass die (BBA)₅, obwohl sie eine höhere Ladungsdichte aufweisen, kein Hydrogel mit Heparin bildeten, wie aus **Tabelle 3** hervorgeht. Dieses Verhalten muss in der Struktur begründet sein, was mit den reinen Peptiden analysiert wurde.

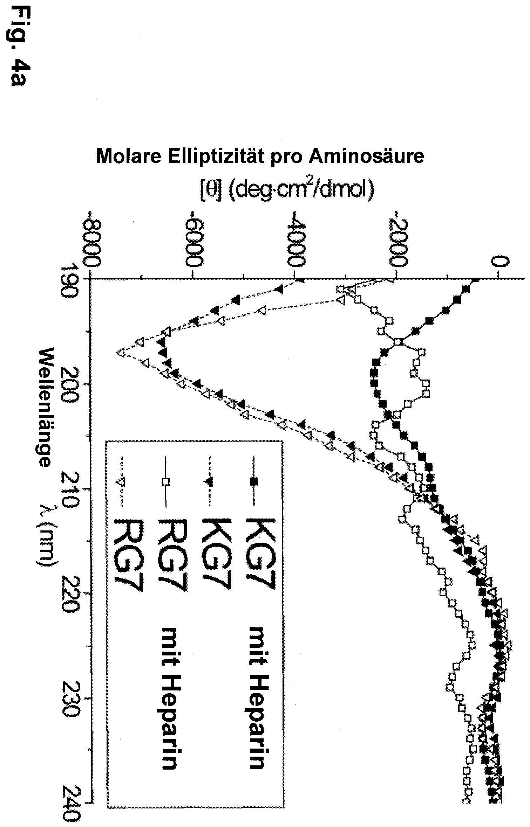

Die *de novo* hergestellten Heparin-bindenden Peptide konnten durch Anwendung der Zirkulardichroismus-Spektroskopie (CD) (J-810, REV. 1.00, JASCO Inc., Easton, MD, USA) analysiert werden. Alle CD-Spektren wurden in einer Quarz-Küvette von 1 mm-Weglänge, bei Wellenlängen von 185 bis 260 nm, aufgenommen. Die Datenpunkte wurden bei jedem Nanometer mit einer Ansprechzeit von 4,0 s aufgetragen. Alle Werte werden als molare Elliptizität [θ], bezogen auf die mittlere Anzahl an Peptidbindungen in deg cm² dmol, gezeigt. Die **Figuren 4a** bis **4f** enthalten das Ergebnis der Analyse der Heparin-abhängigen Strukturänderung durch die Zirkulardichroismus-Spektroskopie (CD). Dabei wurden Peptide in MilliQ-Wasser allein und zusammen mit 14 kDa Heparin in einem Molverhältnis von 1 : 1 gemessen. Nur für RA7 und KA7 wurde doppelt so viel Heparin wie Peptid verwendet. Der Graph für die Peptide, die mit 14-kDa-Heparin gemischt wurden, wurde mit den CD-Spektren von reinem 14-kDa-Heparin unter Nutzung der gleichen Konzentration korrigiert. Der Bereich für die Peptid-Konzentration lag bei 74,5 µM bis 137,6 µM.

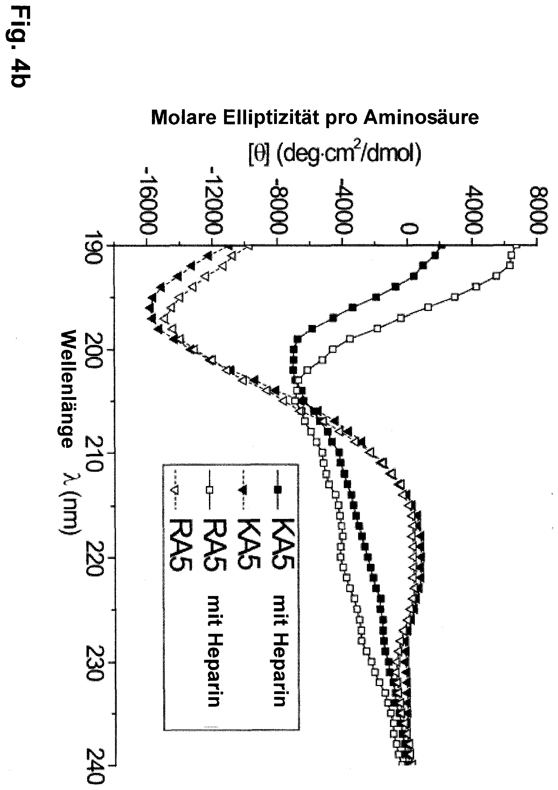

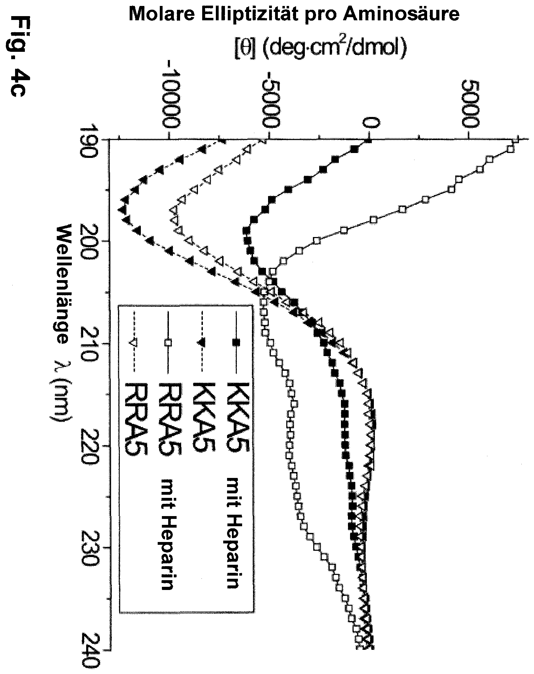

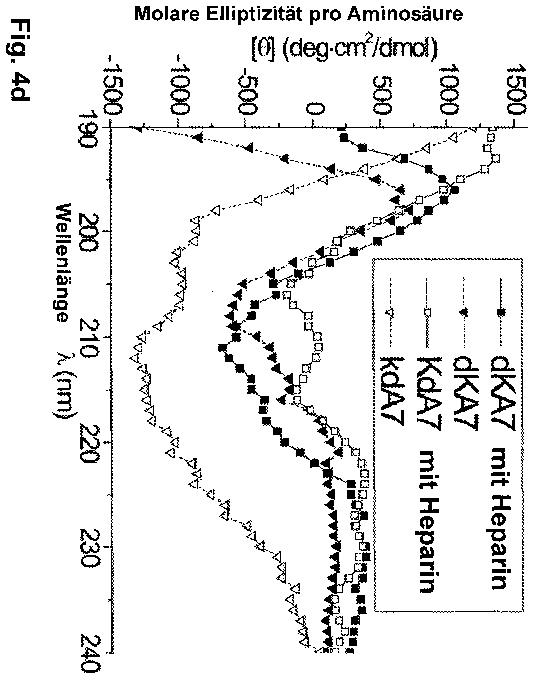

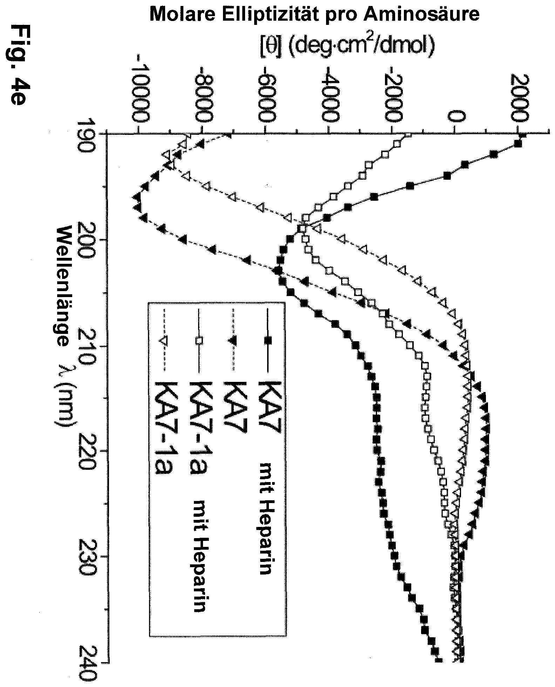

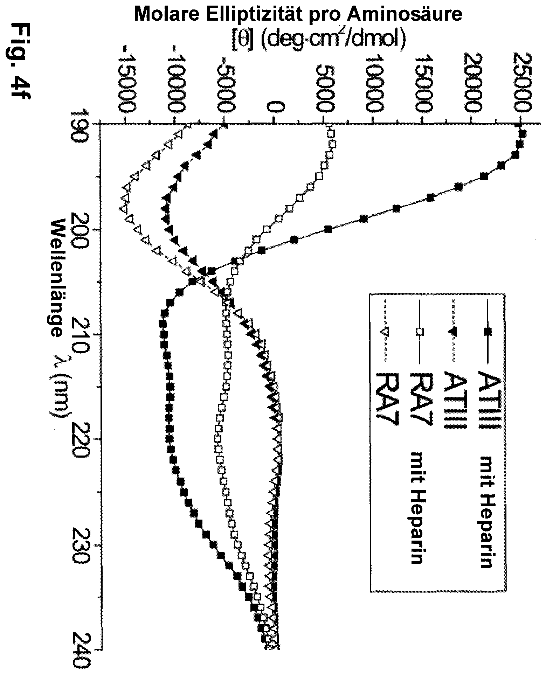

In Milli-Q-Wasser (Advantage A10; Millipore GmbH) zeigten nicht nur (BG)ₙ- und (BBG)₅-Motive eine Zufallsknäuel-Struktur, sondern auch (BA)ₙ- und (BBA)₅-Motive, wie die **Figuren 4** **a** bis **4f** zeigen. Während der Beobachtung der Bildung von Hydrogelen der Peptid-Stern-PEG-Konjugate mit Heparin zeigten die (BA)₇-Motive und die (BA)₅-Motive zusammen mit Heparin eine Strukturänderung. Aufgrund der Glycine können (BG)ₙ-Motive und (BBG)₅-Motive nicht in signifikantem Umfang die Struktur ändern, was sich auch in der fehlenden Hydrogel-Bildung ausdrückt. RRA7 ist das einzige Peptid, das mit Heparin eine Strukturänderung in der Zirculardichroismus-Spektroskopie (CD) zeigte, jedoch keine Hydrogelbildung durchlief. Aufgrund der dichteren Ladungsverteilung ist die optimale Entfernung der positiven Ladung des Peptids für eine optimale Wechselwirkung mit dem Sulfat des Heparins nicht gegeben. Das (BA)ₙ-Peptidmotiv ist daher für Heparin-bindende Peptide bevorzugt, obwohl die (BBA)₅-Peptidmotive mehr positive Ladung bei ähnlicher Peptidlänge aufweisen. Diese Struktur-Aktivitäts-Beziehung zwischen dem Abstand von den basischen Aminosäuren zu Alanin und der Heparinbindungsfähigkeit in Form der Bildung von Hydrogelen ist in jedem Fall neu und überraschend.

Um schließlich die Bedeutung der Struktur-Aktivitäts-Beziehung von (BA)ₙ-Motiven zu unterstreichen, wurden die Mutanten dKA7 und KdA7 synthetisiert, die auch in der **Tabelle 3** aufgeführt sind. Durch die Mischung von L- und D-Aminosäuren sollte die Strukturbildung gestört werden. Die Analyse zeigte, dass diese Mutanten weder ein an Stern-PEG gekoppeltes und mit 14-kDa-Heparin gemischtes Hydrogel bilden (siehe **Tabelle 3)** noch einer von diesen Mutanten eine Strukturänderung ähnlich wie die KA7 zeigt, was auch dem Vergleich der **Figuren 4d** und **4e** zu entnehmen ist. Auch der Mutant KA7-1a mit Austausch von einem L-Alanin durch ein D-Alanin, wobei in der Mitte des Peptid-Motivs bildet kein Hydrogel, das mit Stern-PEG gekoppelt und mit Heparin gemischt ist, wie ebenfalls aus der **Tabelle 3** hervorgeht. Auch tritt nach Zugabe von Heparin keine Strukturänderung im Zirculardichroismus (CD) auf, wie aus der **Fig. 4e** deutlich wird.

Es wurde eine vergleichende Hochdurchsatzanalyse bezüglich der mechanischen Eigenschaften der Hydrogele durchgeführt. Um eine schnelle Hochdurchsatz-Methode für den Vergleich kleiner Mengen von Hydrogel zur Verfügung zu haben, wurde eine Tischzentrifuge (5424R, Eppendorf, Hamburg, Deutschland) verwendet. Dafür wurden 50 µl des Hydrogels durch Mischung der Peptid-Stern-PEG-Konjugate und 14-kDa-Heparin in physiologischem Phosphatpuffer (1XPBS, pH 7,4) bis zu einer Endkonzentration von 5 mM (einmal 2,5 mM für KA7-Stern-PEG) gebildet. Das Gemisch wurde in 0,2 ml Reaktionsgefäßen über Nacht inkubiert. Die Deformation der Hydrogeloberfläche wurde, bezogen auf den 45 °-Zentrifugenrotor und das Eindringen von 275 µm Metallkugeln abhängig von der Kraft, die durch die Zentrifuge aufgewendet werden muss, bestimmt. Alle Experimente wurden dreifach wiederholt.

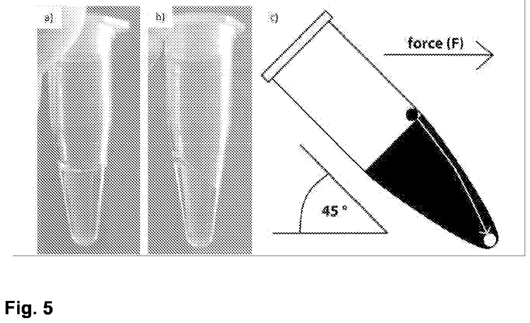

Die **Fig. 5** stellt die Hochdurchsatzanalyse bezüglich der mechanischen Eigenschaften der Hydrogele schematisch dar. Dabei zeigen **a)** und **b)** eine Deformation der Oberfläche des 50 µl Hydrogels in einer 0,2 ml Reaktionstube, genauer gesagt **a)** unterhalb der Geschwindigkeit, die benötigt wird, um die Oberfläche zu deformieren, und **b)** bei der Geschwindigkeit, die nötig ist, um die Oberfläche zu deformieren. Der Teil **c)** der **Fig. 5** ist eine schematische Darstellung des Eindringens eines Kügelchens durch 50 µl Hydrogel in einer 0,2 ml Reaktionstube, abhängig von der Kraft, die durch die 45 °-Zentrifuge angewendet wird.

Es stellte sich heraus, dass das RA5-Stern-PEG-Hydrogel mit Heparin ähnliche Ergebnisse hervorbrachte wie die Mischungen mit RRA7-Stern-PEG, die kein Hydrogel gebildet hatten, siehe **Tabellen 2** und **3.** Die RA7-, KA5- und KA7-basierten Hydrogele sind viel stärker und zeigen gemäß **Tabelle 2** ein breites Spektrum an Steifigkeit. Arginin und Lysin haben unterschiedliche Ladungsverteilungen an der Seitenkette, was zu verschiedenen Hydrogel-Eigenschaften führt. Die zwei verschiedenen Konzentrationen von KA7-Stern-PEG mit Heparin ergaben Hydrogele mit unterschiedlichen mechanischen Eigenschaften, wie in **Tabelle 2** zu erkennen ist. Dies zeigt, dass das auf einer nichtkovalenten Peptid-Biomolekül-Wechselwirkung basierende Hydrogel über verschiedene Wege eingestellt werden kann. Dabei ist es möglich, mit der Konzentration der Komponenten und mit der Peptidsequenz zu experimentieren. Mischungen verschiedener (BA)ₙ-Peptid-Motive auf ein Stern-PEG-Molekül oder verschiedene Peptid-Stern-PEG-Konjugate würden sogar noch weiter die Fähigkeit zur Einstellung in kleineren Schritten verbessern.

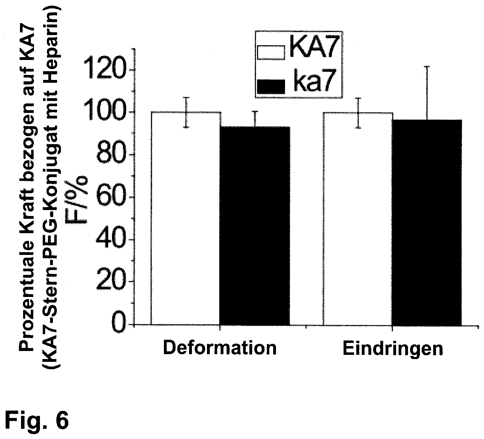

Die **Fig. 6** zeigt die Stabilität eines auf Lysin und Alanin basierenden Hydrogels mit Vergleich von L- und D-Aminosäuren. Die Hydrogele wurden durch Mischen der Endkonzentrationen von 5 mM Peptid-Stern-PEG-Konjugat und 5 mM 14-kDa-Heparin in 50 µl physiologischer Phosphatpufferlösung (1XPBS) gebildet. Die Analyse wurde durch Zentrifugieren der Hydrogele in einer 45 °-Tischzentrifuge mit 275 µm Metallkugeln auf der Gel-Oberfläche durchgeführt. Die Verformung der Oberfläche und das Eindringen der Kugeln wurden in Abhängigkeit von der aufgebrachten Kraft aufgenommen.

Als wichtiges Ergebnis stellte sich heraus, dass die komplette Änderung der KA7 zu D-Aminosäuren nichts an der Hydrogel-Steifigkeit ändert, wie **Fig. 6** zeigt. Aufgrund der Resistenz von D-Aminosäuren gegen Proteasen ist es möglich, nichtkovalente Hydrogele zu schaffen, die in biologischen Umgebungen sehr stabil sind. Auf diese Weise kann die Abbaubarkeit durch unterschiedliche Aminosäuren eingestellt werden.

Um das breite Spektrum der mechanischen Eigenschaften gemäß **Tabelle 2** zu überprüfen, erfolgte eine rheologische Untersuchung, das heißt, es wurde das Fließverhalten der KA7-Stern-PEG- und der KA5-Stern-PEG-Hydrogele mit Heparin über Frequenzdurchlauf- und Belastungsabtastungsexperimente ermittelt.

Die **Fig. 7a** zeigt den Amplitudenverlauf des reinen Peptid-Stern-PEG-Konjugats und des reinen 14-kDa-Heparins mit einer Frequenz von 1 Hz. Die **Fig. 7b** zeigt den Frequenzverlauf des reinen Peptid-Stern-PEG-Konjugats und des reinen 14-kDa-Heparins mit 2 % Amplitude. Die **Figuren 7c** und **7d** stellen das Fließverhalten von Peptid-Stern-PEG-Konjugat als Gemisch mit 14-kDa-Heparin dar. Die Endmischung in physiologischer Phosphatpufferlösung (1XPBS) enthält von beidem 5 mM oder 2,5 mM Peptid-Stern-PEG-Konjugat und 5 mM oder 0,5 mM Heparin. Die Lösungen beziehungsweise Gemische wurden unter Anwendung eines schubspannungsgesteuerten Rheometers (MCR 301, Paar Physica, Anton Paar, Ashland, VA) bei 20 °C und einer Messeinheit mit 39,979 mm Durchmesser, einem Winkel von 0,305° und einer Trunkierung von 24 µm analysiert.

Die Einzelkomponentenlösungen von 14-kDa-Heparin, KA5-Stern-PEG und KA7-Stern-PEG wurden analysiert, um die mechanischen Basiseigenschaften der Ausgangskomponenten im Vergleich zu den mechanischen Eigenschaften der Gemische zu zeigen. Die Einzelkomponenten-Peptid-Stern-PEG-Lösungen wurden exakt genauso behandelt wie die Gemische, die 14-kDa-Heparin enthielten. Alle Gemische und Lösungen wurden unter Anwendung einer durchgehend geschlossenen Umgebung um die Messeinheit herum inkubiert, um ein Verdampfen zu verhindern. Alle Inkubationszeiten wurden durch ein später beschriebenes Gelierungszeitexperiment ermittelt. Das Endgemisch von 5 mM KA7-Stern-PEG und 5 mM 14-kDa-Heparin wurde 1,5 Stunden lang inkubiert. Das Endgemisch von 2,5 mM KA7-Stern-PEG und 0,5 mM 14-kDa-Heparin wurde 3 Stunden lang inkubiert. Das Endgemisch von 5 mM KA5-Stern-PEG und 5 mM 14-kDa-Heparin wurde 15 Stunden lang inkubiert. Die Amplitudenverlaufsmessungen wurden mit einer Frequenz von 1 Hz über einen Bereich von 0,1 bis 100 % durchgeführt. Die Frequenzabhängigkeiten wurden unter Anwendung einer 1 % Amplitude und in einem Bereich von 0,01 bis 100 Hz erfasst. Alle Experimente wurden zweifach wiederholt und der Mittelwert aufgetragen.

Der Speichermodul G' war für alle Proben signifikant höher als der Verlustmodul G" (∼ 2%). Diese viskoelastischen Eigenschaften bestätigen, dass die Wechselwirkung zwischen dem (KA)ₙ und dem Heparin sehr stark und stabil ist. Die Steifigkeit des reinen 14 kDa-Heparins oder des reinen Peptid-Stern-Konjugates ist sehr gering, wie die **Figuren 7a** und **7b** zeigen. Der weite Konzentrations-Bereich von Heparin, welcher verwendet werden kann, reicht von 0,5 bis 5 mM. Auch die mechanischen Eigenschaften können mit einem Faktor von mehr als 10 allein durch Änderung der Konzentration der Komponenten eingestellt werden. Das Mischen unterschiedlicher Peptid-Stern-PEG-Konjugate ist ein zusätzlicher Weg, um die Gel-Eigenschaften zu ändern. Damit stehen zwei Dimensionen zur Verfügung, die Konzentration und die Peptidsequenz, die einzeln oder gemeinsam verändert werden können, um Hydrogel-Eigenschaften für jeden Bedarf anzupassen. Dies ist allein durch die Wechselwirkung des Peptid-Motivs (BA)ₙ mit dem Biomolekül Heparin möglich.

Um die Stärke der Wechselwirkung zwischen dem Peptid-Motiv und dem 14-kDa-Heparin zu überprüfen, wurden die gebildeten Hydrogele gegenüber verschiedenen Lösungsmitteln getestet. Die **Tabelle 4** stellt das Ergebnis dieses Stabilitätstests gegen verschiedene Lösungsmittel dar. Dafür wurden die Peptid-Stern-PEG-Konjugate mit 14-kDa-Heparin in 50 µl physiologischer Phosphatpufferlösung (1XPBS) zu einer Endkonzentration von jeweils 5 mM gemischt. Jedes Lösungsmittel, das heißt physiologische Phosphatpufferlösung (1XPBS), Milli-Q-Wasser, 1 M Salzsäure (HCl), 1 M Natriumhydroxidlösung (NaOH), gesättigte Kochsalzlösung (NaCl), Dimethylsulfoxid (DMSO), Ethanol und Zellkulturmedium mit 2 % fetalem Rinderserum (FBS), wurde jeweils als 200 µl-Überstand dem Hydrogel hinzugegeben. Das Hydrogel wurde bei einer Raumtemperatur von 24 °C inkubiert und die Überstände wurden mindestens drei Tage hintereinander jeden Tag gewechselt. Alle Experimente wurden dreifach durchgeführt. Unter keiner der getesteten Bedingungen konnte das KA7-Hydrogel zerstört werden. Kein anderes bekanntes nichtkovalentes Heparin-abhängiges Hydrogel ist derart stabil, was die außerordentlich stabile Wechselwirkung zwischen dem KA7 und dem Heparin unterstreicht. Selbst das Hydrogel auf der Basis von sehr kurzem KA5 konnte von 1M HCl erst nach mehr als einer Woche Inkubation zerstört werden. 2,2,2-Trifluorethanol (TFE) ist dafür bekannt, jede Art von sekundären Strukturen zu zerstören. Obwohl KA7-Stern-PEG-Hydrogel mit Heparin unzerstörbar scheint, kann die Struktur durch Hinzufügen von 2,2,2-Trifluorethanol (TFE) zum Überstand zerstört werden. Die Gefriertrocknung dieser 2,2,2-Trifluorethanol (TFE)-, KA7-Stern-PEG-, Heparin-Lösung und die Zugabe von physiologischer Phosphatpufferlösung (1XPBS) führte wiederum zu einem klaren Gel.

**Tabelle 4**

| Überstand | KA7 | KA5 | RA7 |
|---|---|---|---|
| 1XPBS | stabil | stabil | stabil; die Oberfläche des Hydrogels wurde milchig |
| Wasser | stabil | stabil | stabil |
| 1 M HCl | stabil | stabil; Hydrogel war erst nach einer Woche zerstört | stabil; Hydrogel wurde milchig und danach wieder klar |
| 1 M NaOH | stabil | stabil | stabil |
| gesättigte NaCl-Lösung | stabil | stabil | nicht stabil; Hydrogel wurde milchig |
| DMSO | stabil | stabil | stabil |
| Ethanol | stabil | stabil | stabil |
| Zellkulturmedium | stabil | stabil | stabil |

Für den Hydrogelbildungsstabilitätstest wurden jeweils drei unterschiedliche Fälle, unter welchen die Hydrogele normalerweise geformt werden, getestet. Die **Tabelle 5** zeigt den Test zur Bildung der Hydrogele in verschiedenen Lösungsmitteln. Die Hydrogele wurden durch Mischung der Endkonzentrationen von jeweils 5 mM Peptid-Stern-PEG-Konjugat und 5 mM 14-kDa-Heparin in 50 µl physiologischer Phosphatpufferlösung (1XPBS), Milli-Q-Wasser oder Zellkultur-Medium mit 2 % fetalem Rinderserum (FBS) gebildet. Die Stabilität der Hydrogele wurde mit dem gleichen Lösungsmittel getestet, in dem es in 200 µl Überstand gebildet wurde. Das Hydrogel wurde bei Raumtemperatur (24 °C) inkubiert, die Überstände wurden mindestens drei Tage hintereinander täglich ausgewechselt und das Ergebnis nach mindestens 3 Tagen ausgewertet.

**Tabelle 5**

| Peptid-Stern-PEG-Konjugate | PBS | Wasser | Zellkulturmedium |
|---|---|---|---|
| KA7 | geformt | geformt | geformt |
| KA5 | geformt | geformt | geformt |
| RA7 | geformt | geformt | geformt |

Zellkulturmedium mit 2 % fetalem Rinderserum (FBS) enthält eine Menge von Proteinen und anderen Komponenten, die gegebenenfalls die Wechselwirkung zwischen dem (BA)ₙ-Peptid-Motiv und 14-kDa-Heparin unterbrechen können, falls sie nicht spezifisch oder stabil genug ist.

Des Weiteren wurde ein Erosionsexperiment durchgeführt. Dazu wurden Peptid-Stern-PEG-Konjugate jeweils mit TAMRA-markiertem 14-kDa-Heparin gemischt. Zuvor musste TAMRA-markiertes 14-kDa-Heparin für das Erosionsexperiment synthetisiert werden. Dabei wurde 14 kDa-Heparin mit 5-(und-6)-Carboxytetramethylrhodamin (TAMRA, Invitrogen) unter Anwendung der 1-Ethyl-3-(3-Dimethylaminopropyl)carbodiimid/N-hydroxysulfosuccinimid-(EDAC/sNHS)-Chemie markiert. Heparin, TAMRA, EDAC, sNHS und Na₂CO₃ wurden in einem Molverhältnis von 1 : 2 : 5 : 4 : 20 in Wasser gemischt und über Nacht inkubiert. Danach wurde das Gemisch unter Anwendung einer Dialysemembran mit einer Ausschlussgrenze von 8 kDa gegen 10 Liter Wasser unter konstantem Wasseraustausch 2 Tage lang dialysiert. Das dialysierte Produkt wurde durch einen 0,22 µm Polyvinylidenfluorid-Filter (PVDF-Filter) filtriert und zu einem roten Produkt gefriergetrocknet.

Schließlich wurden die Peptid-Stern-PEG-Konjugate jeweils mit dem TAMRAmarkierten 14-kDa-Heparin in 50 µl Zellkulturmedium mit 2 % fetalem Rinderserum (FBS) bis zu einer Endkonzentration von 5 mM gemischt. Die Hydrogele wurden bei 37 °C bei 95 % Luftfeuchtigkeit und 5 % CO₂ über Nacht (15 Stunden) (Galaxy 170S, Eppendorf, Hamburg, Germany) gebildet. Nach einer Inkubation über Nacht wurde 1 ml des Zellkultur-Mediums mit 2 % fetalem Rinderserum (FBS) hinzugegeben. Es wurden 200 µl des Überstands zu jedem Messzeitpunkt entnommen und mit neuem Zellkultur-Medium mit 2 % fetalem Rinderserum (FBS) ausgetauscht. Die Fluoreszenz wurde zu bestimmten Zeitpunkten im Überstand, unter Nutzung eines Plattenlesegeräts (BECKMAN COULTER PARADIGM Detektionsplattform, BECKMAN COULTER, Brea, California, USA) und von schwarzen 96-Well-Platten mit klarem Boden, gemessen.

Die **Fig. 8a** zeigt zunächst die Analyse einer Heparin-abhängigen Strukturänderung durch Zirkulardichroismus-Spektroskopie. Beide Peptide zeigten ohne Heparin in Milli-Q-Wasser eine Zufallsknäuelstruktur. Nach Zugabe von 14-kDa-Heparin in einer 2-molaren Konzentration der Peptide trat eine klare Strukturänderung auf. Die **Fig. 8b** zeigt schließlich das Ergebnis der Überprüfung der Erosion des Hydrogels durch das oben erwähnte Zusammenmischen von Peptid-Stern-PEG-Konjugat und TAMRA-markiertem 14-kDa-Heparin in 50 µl Zellkulturmedium mit 2 % fetalem Rinderserum (FBS) zu einer Endkonzentration von 5 mM. Die Fluoreszenz wurde in 200 µl von 1 ml Überstand gemessen. Diese 200 µl wurden jeweils durch 200 µl frisches Medium ersetzt.

Wie aus der Literatur, unter anderem aus JR Fromm, RE Hileman, EBO Caldwell, JM Weiler, RJ Linhardt, Archives of Biochemistry and Biophysics 1997, 343, 92-100**,** bekannt ist, bindet Arginin stärker als Lysin an Heparin. RA7 ist stärker an Heparin gebunden, so dass weniger Heparin aus dem Hydrogel mit RA7-Stern-PEG freigegeben wird als aus dem Hydrogel mit KA7-Stern-PEG. KA5 hat weniger Ladung als KA7, so dass die Bindung geringer ist, was zu mehr Erosion führt. Die Hydrogele verloren vernachlässigbar an Masse, so dass es wahrscheinlich ist, dass das meiste Heparin, das freigesetzt wurde, das heißt bis zu 35 %, nicht Teil des Hydrogel-Netzwerks ist. Nach der Stabilisierung der Heparin-Erosion aus dem Hydrogel ist dieses stabiler als Protein-Hydrogele. Die Tatsache, dass das Hydrogel weiterhin sehr stabil gegenüber Serum und seinen Komponenten ist, zeigt die Spezifität der Wechselwirkung zwischen dem (BA)ₙ-Peptid-Motiv und dem Heparin. Damit muss das Hydrogel vor der Anwendung nicht vorgebildet werden. Dies ist ein sehr großer Vorteil, da dies Zeit spart und die Konzentration, zum Beispiel der Proteine, gleichmäßig verteilt wird. Zudem ist die Reproduzierbarkeit größer, da es weniger Herstellungsschritte gibt.

Für eine Rasterelektronen-mikroskopische Aufnahme wurden KA7-Stern-PEG-Konjugate mit 14-kDa-Heparin zu einer Endkonzentration von 5 mM jeweils in 50 µl physiologischer Phosphatpufferlösung (1XPBS) gemischt und 3 Tage lang bei Raumtemperatur inkubiert. Die Probe wurde durch Schieben einer Kapillartube in das Gel, Schockfrieren in flüssigem Stickstoff und Schneiden durch die Probe mit einem sehr scharfen Messer genommen. Die Oberflächengetrocknete und geschnittene Probe wurde mit einem Rasterelektronenmikroskop bildlich aufgenommen (Supra 40VP, Zeiss, Jena, Deutschland).

Die **Fig. 9** zeigt eine Raster-Mikroskopie-Abbildung des KA7-Stern-PEG-Hydrogels mit Heparin. Die Probe wurde in flüssigem Stickstoff schockgefroren und nach einer kurzen Zeit der Verdunstung analysiert. Das KA7-Stern-PEG-Hydrogel zeigte eine deutliche Netzwerkstruktur.

Im Zusammenhang mit der Hydrogel-Präparation ist die Gelierungszeit wichtig. Anfangs sollte die Gelierungszeit durch Nutzung des schubspannungsgesteuerten Rheometers (MCR 301, Paar Physica, Anton Paar, Ashland, VA) bei 20 °C und einer Messeinheit mit 39,979 mm Durchmesser, einem Winkel von 0,305° und einer Trunkierung von 24 µm bestimmt werden. Unvorteilhafterweise änderte die Messung mit 2 % Amplitude und einer Frequenz von 1 Hz die Gelierungszeit. Die Gelierung trat viel schneller auf als zuvor im Labor beobachtet. Aufgrund dieses Verhaltens gab es eine Notwendigkeit für einen alternativen Weg, die Gelierungszeit zu messen. Es wurde eine Mikrochip-gesteuerte Maschine gebaut und programmiert, die in der Lage ist, die zeitabhängige Hydrogel-Steifigkeit auf einer Feinwaage (XP 205 Feingewicht Delta Range, Mettler-Toledo GmbH, Gießen, Deutschland) zu messen. Die **Fig. 10** zeigt eine Einrichtung zur Analyse der Gelierungszeit eines Hydrogels, gesteuert durch einen programmierbaren Mikrochip, und die Nutzung der Feinwaage zur Messung der Gelierungszeit der Hydrogele. Gezeigt ist ein beweglicher Teil auf der Feinwaage, bestehend aus einer stumpfen Nadel und einem Halter für ein 0,2 ml Reaktiongefäß, welches die Hydrogelmischung enthält. Eine LabX-Software, die auf einem Computer installiert war, wurde genutzt, um die Kraft zu überwachen und aufzunehmen.

Unterschiedliche Konzentrationen von Peptid-Stern-PEG-Konjugaten wurden während konstanter Verwirbelung mit 14-kDa-Heparin gemischt, um 50 µl Hydrogel in physiologischer Phosphatpufferlösung (1XPBS) in dem 0,2 ml-Reaktionsgefäß zu bilden. Nach dem Mischen wurde das Reaktionsgefäß mit einem Deckel mit einem 1,5 mm Loch verschlossen und die Messung umgehend gestartet. An der Innenseite des Deckels schützten 10 µl Wasser die Hydrogeloberfläche vor dem Austrocknen. Zu Beginn der Messung befindet sich die stumpfe Nadel von 1 mm Durchmesser von oben betrachtet 1 mm tief im Hydrogel. Aller 5 Minuten bewegt sich die stumpfe Nadel 1 mm in das Gel und wird nach einer Sekunde Wartezeit wieder bis 30 µm unterhalb der Originalposition nach oben bewegt. Diese 30 µm Höhendifferenz stellt sicher, dass die Nadel keinen Kanal im Gel erzeugt, welcher keinerlei Widerstand aufweist, aber geht jede Messung 30 µm tiefer und tiefer in das Gel (geradeaus von oben), um immer ein unberührtes Hydrogelgemisch vorliegen zu haben, welches gemessen werden kann. Alle Daten von der Feinwaage wurden überwacht und dokumentiert unter Nutzung der LabX Software (Mettler-Toledo GmbH, Gießen, Deutschland), die auf einem Notebook installiert und mit einer RS-232-Serienverbindung mit der Feinwaage verbunden ist. Die Amplituden des Widerstandes des Hydrogels gegen den Druck nach dem Herunterdrücken der Nadel, korrigiert durch die Basislinie vor der Aufnahme eines Messzeitpunktes, wurden aufgetragen.

Die **Figuren 11a** bis **11c** stellen dabei das Ergebnis der Analyse der Gelierungszeit des Hydrogels durch Messen der Kraft dar, die benötigt wird, um unter Verwendung einer Nadel mit 1 mm Durchmesser einzudringen. Unmittelbar nach dem Mischen der Komponenten wurde die Messung alle 5 Minuten durchgeführt. Für jede Messung wurde die Nadel 1 mm in der Mischung nach unten und 0,970 mm nach oben bewegt. Die Kraft wurde durch Wiegen gemessen und die Amplitude, korrigiert durch die Basislinie, aufgetragen.

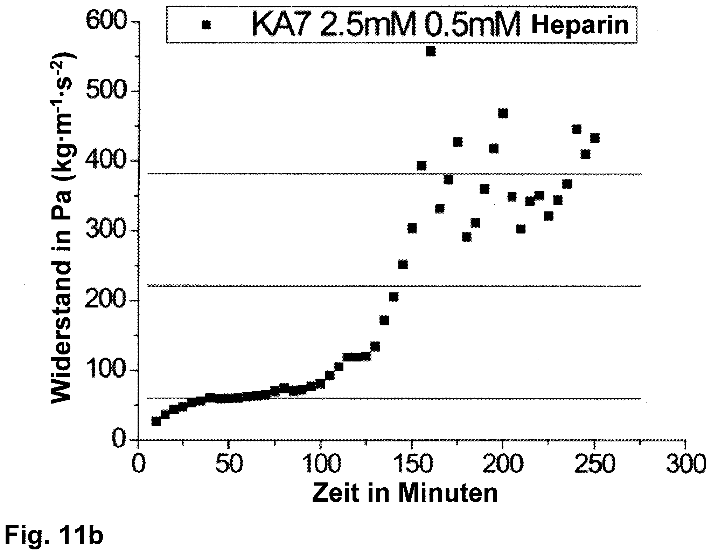

Aufgrund der unterschiedlichen Ladungseigenschaften von RA7-Stern-PEG, KA7-Stern-PEG und KA5-Stern-PEG unterscheidet sich die Gelierungzeit. RA7-Stern-PEG-Hydrogel bildet sich mit 14-kDa-Heparin in physiologischem Phosphatpuffer (1XPBS) sofort mit einer Endkonzentration von 5 mM. KA7-Stern-PEG braucht für die Bildung des Hydrogels unter den gleichen Bedingungen etwa eine Stunde und KA5-Stern-PEG mehrere Stunden. Durch die Absenkung der Konzentration der Komponenten erhöht sich die Gelierungszeit, wie ein Vergleich der **Figuren 11a** und **11b** zeigt. Das Mischen von verschiedenen (BA)ₙ-Peptid-Motiven, die an Stern-PEG gekoppelt sind, würde eine Möglichkeit eröffnen, Steifigkeit und Gelierungszeit zusammen einzustellen. Dies gibt dem Nutzer ein System in die Hand, mit dem die Gelierungszeit durch Änderung der Konzentration der Komponenten oder des Verhältnisses der verschiedenen an Stern-PEG gekoppelten (BA)ₙ-Peptid-Motive bei Beibehaltung des Feststoffgehaltes eingestellt werden kann.

Das Hydrogel, bestehend aus den (BA)ₙ-Stern-PEG-Konjugaten mit Heparin, ist nicht toxisch gegenüber Säugetier-Zellen, wie in einem *in vitro-*Zytotoxizitätstest der Hydrogelkomponenten gezeigt werden konnte (siehe **Figuren 12a** bis **12f****.** Da Fibroblasten der wichtigste Bestandteil aller Bindegewebe sind, wurden sie erfolgreich für eine 9-tägige 3D-Zellkultivierung unter Anwendung von KA7-Stern-PEG oder KA5-Stern-PEG mit Heparin in Zellkultur-Medium mit 2 % fetalem Rinderserum (FBS) genutzt.

Ein gefrorenes Fläschchen mit Zellen wurde in einem 37 °C warmen Wasserbad für 2 Minuten aufgetaut. Die Zellen wurden immer in 5 ml vollständiges Zellkulturmedium 106 (mit 2 % fetalem Rinderserum (FBS)) übertragen. Diese Zellsuspension wurde bei 700 g in einer Zentrifuge (ROTINA 380 R, Hettich, Tuttlingen, Deutschland) zentrifugiert, der Überstand entfernt und die Zellen in 6 ml vollständigem Zellkulturmedium resuspendiert. Nach dem Mischen wurde die Suspension in ein Zellkulturgefäß übertragen und bei 37 °C, 95 % Luftfeuchtigkeit und 5 % CO₂ inkubiert. Aller 2 Tage wurde das Medium gewechselt, bis die Zellen konfluent waren. Das Zellkulturmedium aus der konfluenten Phase der Zellen wurde entfernt und 1 ml Typsin/EDTA-Lösung wurde der Zellschicht hinzugefügt. Nach 5 Minuten und gelegentlichem Rütteln sind die Zellen in Suspension und es wurden 3 ml des neuen vollständigen Zellkulturmediums dazugegeben. Diese 4 ml Zellsuspension wurden verdünnt in 20 ml des vollständigen Zellkulturmediums und in 4 neue Zellkulturgefäße (jedes 6 ml) übertragen. Dann wurden sie weiter, wie beschrieben, bis zur Nutzung oder weiteren Aufteilung inkubiert.

Das Zellkulturmedium aus der konfluenten Schicht der Zellen wurde entfernt und 1 ml der Typsin/EDTA-Lösung wurde zu der Zellschicht hinzugefügt. Nach 5 Minuten und gelegentlichem Schütteln sind die Zellen in Suspension und es wurden 3 ml des neuen vollständigen Zellkulturmediums hinzugefügt. Die Menge der Zellen wurde durch Mischung von 50 µl der Zellsuspension mit 50 µl der Trypan-Blaulösung gezählt, wobei die Anzahl der Zellen mit einem Hämocytometer erhalten wurde. Die 4 ml der Zellsuspension wurden bei 700 g in einer Zentrifuge (ROTINA 380 R, Hettich, Tuttlingen, Deutschland) zentrifugiert, der Überstand entfernt und die Zellen in einer Menge von vollständigem Zellkulturmedium gelöst, um die Zielkonzentration zu erreichen. Die Untersuchungen zur Toxizität der Peptid-Stern-PEG-Konjugate wurden durchgeführt durch Impfen von 5000 HDFn-Zellen pro Well in einer 96-Well-Platte. Nach dem Transfer der Zellen waren diese in der Lage, sich an die 96-Well-Platten anzuheften. Die Zellen wurden 24 Stunden vor der Verabreichung der Proben bei 37 °C, 5 % CO₂ und 95 % Luftfeuchte inkubiert.

Die **Figuren 12a** bis **12f** zeigen einen Toxizitätstest für verschiedene Peptid-Stern-PEG-Konjugate und 14-kDa-Heparin. Das Zellmedium wurde nach den 24 Stunden Inkubation durch 200 µl einer Lösung ersetzt, die frisches Medium und 10⁻⁴ oder 10⁻⁵ M Peptid-Stern-PEG-Konjugat enthält, welches durch 0,22 µm Zentrifugenröhrchenfilter gefiltert war. Es wurden somit 10⁻⁴ und 10⁻⁵ M Peptid-Stern-PEG-Konjugat oder Heparin zu den 5000 resuspendierten menschlichen Fibroblasten in Zellkulturmedium mit Serum hinzugegeben. Nach der Hinzugabe der gesamten Testproben wurden die Zellen dann weitere 24 Stunden bei 37 °C, 5 % CO2 und 95 % Luftfeuchte inkubiert, um sowohl die zeitabhängige als auch die konzentrationsabhängige Zytotoxizität zu analysieren. Am Ende jeder Aussetzung wurde die Toxizitätsstufe von jeder Testprobe durch einen Test mit 3-(4,5-Dimethylazol-2-yl)-2,5-Diphenyltetrazoliumbromid (MTT) bewertet, um die Zytotoxizität der Peptid-Stern-PEG-Konjugate im Vergleich mit nicht behandelten Zellen zu bestimmen. Dabei wird in lebenden Zellen das gelbe 3-(4,5-Dimethylthiazol-2-yl)-2,5-diphenyltetrazoliumbromid (MTT) zum lilafarbenen Formazan reduziert. Auf die gleiche Weise wurden alle Schritte ohne Zugabe der Verbindung zu den Zellen durchgeführt. Somit wurden die Zellen, die mit Medium allein inkubiert wurden, als Kontrolle genutzt.

Der MTT-Test hilft bei der Beurteilung der Lebensfähigkeit der Zellen durch die Messung der enzymatischen Reduktion von gelbem Tetrazolium zu violetten Formazan-Kristallen. Nach der Inkubation der Zellen mit den Testproben wurde MTT hinzugegeben und weitere 4 Stunden inkubiert. Nach 4 Stunden wurde das Medium entfernt und es wurden 100 µl Dimethylsulfoxid (DMSO) hinzugegeben, um die Formazankristalle zu lösen, die aus der Reduktion des Tetrazoliumsalzes allein durch die metabolisch aktiven Zellen entstanden waren. Die Absorption der gelösten Formazankristalle wurde bei 570 nm unter Nutzung eines Plattenlesers gemessen (BECKMAN COULTER PARADIGM Detection platform, BECKMAN COULTER, Brea, California, USA). Da die Absorption direkt die Zahl der lebensfähigen Zellen anzeigt, wurde die prozentuale Lebensfähigkeit direkt aus den Absorptionswerten errechnet. Die Durchschnittstoxizität wurde durch den Mittelwert von 15 Wells der Zellen berechnet, die mit der gleichen Verbindung behandelt wurden.

Die **Figuren 12a** bis **12f** zeigen die Ergebnisse der MTT-Tests für **a)** 14-kDa-Heparin, **b)** ATIII-Stern-PEG-Konjugat, **c)** KA5-Stern-PEG-Konjugat, **d)** RA5-Stern-PEG-Konjugat, **e)** KA7-Stern-PEG-Konjugat und **f)** RA7-Stern-PEG-Konjugat.

Zur Einbettung der Zellen in das Hydrogel wurde zunächst das Zellkulturmedium aus der konfluenten Schicht der Zellen entfernt und dann 1 ml Trypsin/EDTA-Lösung zur Zellschicht hinzugegeben. Nach 5 Minuten und gelegentlichem Schütteln sind die Zellen in Suspension, worauf 3 ml von neuem Zellkulturmedium hinzugegeben wurden. Die Menge der Zellen wurde durch Mischung von 50 µl der Zellsuspension mit 50 µl von Trypan-BlauLösung gezählt, wobei die Zellen unter Nutzung eines Hämocytometers gezählt wurden. Die 4 ml Zellsuspension wurden bei 700 g in einer Zentrifuge (ROTINA 380 R, Hettich, Tuttlingen, Deutschland) zentrifugiert, der Überstand entfernt und die Zellen in einer Menge von vollständigem Zellkulturmedium gelöst, um die Zielkonzentration zu erreichen. KA7-Stern-PEG-Konjugate wurden im gesamten Zellkulturmedium gelöst und durch einen 0,22 µm-Zentrifugenröhrchenfilter filtriert. Das Gleiche wurde mit 14-kDa-Heparin getan. Zur Lösung von KA7-Stern-PEG-Konjugat wurden entsprechend Zellen hinzugefügt, um eine Endkonzentration von 10⁶ Zellen pro Milliliter zu erhalten. Danach wurde die KA7-Stern-PEG-Konjugat-Zell-Mischung mit 14-kDa-Heparin zu einer Endkonzentration von 5 mM von beidem in 50 µl gemischt, auf den Boden einer 8-Well-Platte pipettiert und über Nacht bei 37 °C, 95 % Luftfeuchte und 5 % CO₂ inkubiert. Nach 1 Tag wurden 0,5 ml des gesamten Zellkulturmediums hinzugefügt (und aller 2 Tage ausgetauscht) und die Zellen bei 37 °C, 95 % Luftfeuchte und 5 % CO₂ weiter inkubiert. Die Einbettung der Zellen in die KA5-Stern-PEG-Konjugat-Mischung mit 14-kDa-Heparin wurde auf die gleiche Weise wie beim KA7-Stern-PEG-Konjugat mit ähnlichen Ergebnissen durchgeführt.

Die Lebensfähigkeit der Zellen wurde durch Zugabe von 50 µl MTT (3-(4,5-dimethylthiazol-2-yl)-2,5-Diphenyltetrazoliumbromid) in die 500 µl des vollen Zellkulturmediums, als Überstand der in das Hydrogel eingebetteten Zellen, bestimmt. Die Zellen wurden weiter bei 37 °C, 95 % Luftfeuchte und 5 % CO₂ inkubiert. Die Zellen wurden nach 1 Stunde Inkubationzeit mit einem Präparationsmikroskop abgebildet.

Die Lebensfähigkeit der Zellen wurde mit einem sogenannten Live/Dead®-Assay untersucht. Die Zellen-enthaltenden Gele wurden zweimal mit physiologischer Phosphatpufferlösung (1XPBS) gespült. Eine Lösung von 10 µM Probidiumjodid (PI) (Molekulare Probes, Invitrogen, Deutschland) und 0,15 µM Fluorescin Diacetat (FDA) (Fluka, Deutschland) in physiologischer Phosphatpufferlösung (1XPBS) wurden für drei Minuten auf die Gele aufgebracht, gefolgt von einer Spülung mit 1XPBS. Die Zellen wurden mit einem konfokalen Mikroskop (Leica SP5, 10x/0,4) abgebildet. Die Abbildungen wurden aller 5 µm für einen Gelabschnitt von 100 µm Dicke aufgenommen und die Maximumintensitätsprojektion (MIP) der Abbildungen wurde dargestellt.

Vor der Immunfärbung wurden die Proben mit 4 % Paraformaldehyd (PFA) 15 Minuten lang bei Raumtemperatur fixiert und in 0,25 % Rinderserumalbumin (BSA) (Sigma-Aldrich, München, Deutschland) und 1 % Triton-X100 (Sigma-Aldrich, München, Germany) in PBS) geblockt. Als Nächstes wurde Phalloidin-CF488 (Biotrend, Deutschland) 5 Minuten lang in Blocking-Puffer angewandt. Danach wurden 0,1 µg/ml 4',6-Diamidino-2-phenylindol (DAPI, Sigma-Aldrich, München, Deutschland) in 1XPBS für 5 Minuten angewandt, gefolgt von 3 x 15 Minuten Waschen mit dem Puffer. Die Proben wurden mit einem konfokalen Mikroskop abgebildet (Leica SP5, 63x/1.4-0.6).

Die **Figur 13** zeigt in **a)** bis **f)** die Ergebnisse des Lebensfähigkeitstests und die Struktur von eingebetteten menschlichen Fibroblasten (HDFn) aus der Haut von Neugeborenen in einem Hydrogel. Das Endgemisch im vollständigen Zellkulturmedium enthält 5 mM KA5-Stern-PEG-Konjugat und 14-kDa-Heparin für die Teile **a)** bis **c)** und 2,5 mM KA7-Stern-PEG-Konjugat und 14-kDa-Heparin für die Teile **d)** und **f)** der **Fig. 13****.** Die Konzentration der Zellen war 10⁶ Zellen pro ml. Die Teile **a)** und **d)** der **Fig. 13** zeigen weitfeldmikroskopische Bilder, Maßstab 1 mm, von mit 3-(4,5-Dimethylthiazol-2-yl)-2,5-diphenyltetrazoliumbromid (MTT) gefärbten HDFn im Hydrogel. Die Teile **b)** und **e)** der **Fig. 13** zeigen Live/Dead® Assay- gefärbte HDFn. Dabei zeigt Teil **b)** der **Fig. 13** einen Anteil der lebenden Zellen von 99±1 %. Die Teile **c)** und **f)** der **Fig. 13** zeigen Actin-Filamente und den Kern von HDFn, gefärbt mit Pholloidin-CF488 und DAPI. Die Teile **b)** und **c)** sowie die Teile **e)** und **f)** der **Fig. 13** zeigen mikroskopische konfokale Laserscanning-Bilder.

Zusammenfassend lässt sich sagen, dass durch die erfindungsgemäße Hydrogelmatrix biologische Funktionen, wie Nicht-Toxizität für menschliche Zellen, Proteinbindung und einstellbare enzymatische Abbaubarkeit zusammen mit flexiblen physikalischen Eigenschaften, wie Einstellbarkeit der Gelierungszeit und Fließverhalten durch Variation der Oligosaccharide und Peptide (Peptid-Stern-PEG-Konjugate) und deren Konzentrationen und weitreichender chemischer Modifizierbarkeit, rein durch nichtkovalente Interaktionen der Hydrogelmatrix-Komponenten ohne jegliche chemische Reaktionen während der Gelformung ermöglicht werden.

Insbesondere Heparin ist ein hoch sulfatiertes Glycosaminoglycan, das Wachstumsfaktoren bindet, die zum Beispiel in der Zellkultivierung verwendet werden. Nichtkovalente Hydrogele, die auf Heparin und Heparin-bindenden Peptiden basieren, sind entwickelt worden, sind aber nicht abstimmbar. *De novo* hergestellte Heparin-bindende Peptide, deren Eigenschaften durch Einstellen der Länge verändert werden kann, lösen dieses Problem. Alle ordnungsgemäß funktionierenden, bisher *de novo* hergestellten Peptide sind über 20 Aminosäuren lang. Dies würde zu Syntheseproblemen führen, wenn zusätzliche Eigenschaften eingeführt werden sollten. Hier wurde die Vielfalt der Eigenschaften des neu gestalteten (BA)ₙ- Peptid-Motivs durch Nutzung einer entworfenen Peptid-Stern-PEG-Bibliothek für nichtkovalente Hydrogelbildung gezeigt. Nicht nur, dass diese Peptide Strukturänderungen nach der Bindung an Heparin durchlaufen, wie für Peptide natürlichen Ursprungs berichtet, es handelt sich auch um die kürzesten künstlichen Heparin-bindenden Peptide, die aus der Literatur bekannt sind. Die Einstellbarkeit des nichtkovalenten Hydrogels mit (BA)ₙ-Stern-PEG-Konjugaten und Heparin ist überraschend. Es ist möglich, die Steifigkeit, die Gelierungszeit und die biologische und chemische Stabilität allein durch Ändern der Länge, der Konzentration oder der Art der Basisaminosäure des (BA)ₙ Peptid-Motivs einzustellen. Auf diese Weise ist es möglich, die Eigenschaften unter Beibehaltung des Feststoffgehaltes zu verändern, oder den Feststoffgehalt zu ändern und die Eigenschaften stabil zu halten.

## Patentansprüche

1. Nichtkovalente selbstorganisierende Hydrogelmatrix für biotechnologische Anwendungen, enthaltend ein kovalentes Polymer-Peptid-Konjugat, wobei das kovalente Polymer-Peptid-Konjugat Konjugate von zwei oder mehr Peptiden umfasst, die an eine Polymerkette gekoppelt sind, und die Peptid-Sequenz ein sich wiederholendes Dipeptid-Motiv (BA)ₙ enthält, worin B eine Aminosäure mit einer positiv geladenen Seitenkette, A Alanin und n eine Zahl von 5 bis 20 ist, **dadurch gekennzeichnet, dass** diese ferner ein hoch negativ geladenes Oligosaccharid umfasst und die Hydrogelmatrix in Form eines Oligosaccharid/Peptid/Polymer-Systems ausgebildet ist, bei dem das Peptid an das Polymer chemisch konjugiert ist und das Hydrogel durch Mischen des Peptid-Polymer-Konjugates und des Oligosaccharides erhältlich ist.

2. Nichtkovalente selbstorganisierende Hydrogelmatrix nach Anspruch 1, **dadurch gekennzeichnet, dass** die Polymerkette aus einem linearen oder mehrarmigen Polyethylenglykol (PEG) gebildet wird.

3. Nichtkovalente selbstorganisierende Hydrogelmatrix nach Anspruch 1 oder 2, **dadurch gekennzeichnet, dass** das hoch negativ geladene Oligosaccharid ein sulfatiertes oder phosphoryliertes Oligosaccharid ist.

4. Nichtkovalente selbstorganisierende Hydrogelmatrix nach Anspruch 3, **dadurch gekennzeichnet, dass** das hoch negativ geladene Oligosaccharid ausgewählt ist aus einer Gruppe von Oligosacchariden, die Heparin, Dextransulfat, α-Cyclodextrinsulfat, β-Cyclodextrinsulfat, γ-Cyclodextrinsulfat, α-Cyclodextrinphosphat, β-Cyclodextrinphosphat und γ-Cyclodextrinphosphat umfasst.

5. Nichtkovalente selbstorganisierende Hydrogelmatrix nach einem der Ansprüche 1 bis 4, **dadurch gekennzeichnet, dass** diese ferner eine unter Licht spaltbare chemische Gruppe zwischen der Polymerkette und der Peptid-Sequenz umfasst, die das sich wiederholende Dipeptid-Motiv (BA)ₙ enthält.

6. Nichtkovalente selbstorganisierende Hydrogelmatrix nach einem der Ansprüche 1 bis 4, **dadurch gekennzeichnet, dass** diese ferner einen pH-empfindlichen chemischen Linker zwischen der Polymerkette und der Peptid-Sequenz umfasst, die das sich wiederholende Dipeptid-Motiv (BA)ₙ enthält.

7. Nichtkovalente selbstorganisierende Hydrogelmatrix nach einem der Ansprüche 1 bis 4, **dadurch gekennzeichnet, dass** diese ferner einen enzymatisch spaltbaren Linker zwischen der Polymerkette und der Peptid-Sequenz umfasst, die das sich wiederholende Dipeptid-Motiv (BA)ₙ enthält.

8. Nichtkovalente selbstorganisierende Hydrogelmatrix nach einem der Ansprüche 1 bis 7, **dadurch gekennzeichnet, dass** diese ein Elastizitätsmodul von mindestens 10 Pa aufweist.

9. Nichtkovalente selbstorganisierende Hydrogelmatrix nach einem der Ansprüche 1 bis 8, **dadurch gekennzeichnet, dass** diese kugelförmig ausgebildet ist.

10. Verbund einer nichtkovalenten selbstorganisierenden Hydrogelmatrix nach einem der Ansprüche 1 bis 9 mit in die kugelförmige oder nicht_kugelförmige Hydrogelmatrix eingebetteten Zellen.

11. Verbund nach Anspruch 10, **dadurch gekennzeichnet, dass** die Zellen aus einer Gruppe ausgewählt werden, die Säugetierzellen, Insektenzellen, Bakterien-Zellen und Hefezellen umfasst.

12. Verwendung eines Verbundes nach Anspruch 10 oder 11 zur Herstellung von Proteinen, wobei das Protein therapeutische monoklonale Antikörper umfasst.

13. Kapsel zur gezielten Freisetzung von therapeutischen Reagenzien, **dadurch gekennzeichnet, dass** die therapeutischen Reagenzien zusammen mit einer nichtkovalenten selbstorganisierenden Hydrogelmatrix nach einem der Ansprüche 1 bis 8 oder zusammen mit einer kugelförmigen nichtkovalenten selbstorganisierenden Hydrogelmatrix nach Anspruch 9 eingekapselt sind.

14. Zusammensetzung aus einer nichtkovalenten selbstorganisierenden Hydrogelmatrix nach einem der Ansprüche 1 bis 8 und therapeutischen Chemikalien und Reagenzien, **dadurch gekennzeichnet, dass** im therapeutischen Hydrogel ein Gradient der Chemikalien und Reagenzien erzeugt ist.

15. Hybridsystem aus einer nicht kugelförmigen nichtkovalenten selbstorganisierenden Hydrogelmatrix nach einem der Ansprüche 1 bis 8 und einer kugelförmigen nichtkovalenten selbstorganisierenden Hydrogelmatrix nach Anspruch 9, **dadurch gekennzeichnet, dass** die nicht kugelförmige nichtkovalente selbstorganisierende Hydrogelmatrix und die kugelförmige nichtkovalente selbstorganisierende Hydrogelmatrix jeweils eine unterschiedliche chemische Zusammensetzung aufweisen und eine Komponente des Hybridsystems durch Bestrahlung mit Licht, durch selektiven chemischen Abbau oder durch enzymatische Verdauung regelbar ist.

## Claims

1. Non-covalent self-assembling hydrogel matrix for biotechnological applications, comprising a covalent polymer-peptide conjugate, whereby the covalent polymer peptide conjugate includes conjugates of two or more peptides that are coupled to a polymer chain and the peptide sequence contains a recurring dipeptide motif (BA)n, whereby B is an amino acid having positively charged side chain, A is alanine and n is an integer between 5 and 20, **characterised in that** it furthermore includes a highly negatively charged oligosaccharide and the hydrogel matrix is arranged in the form of an oligosaccharide/peptide/polymer-system in which the peptide is chemically conjugated to the polymer and the gel formation is obtained through mixing the peptide-polymer-conjugate and the oligosaccharide.

2. Non-covalent self-assembling hydrogel matrix according to claim 1, **characterised in that** the polymer chain is formed by a linear or multi-arm polyethyleneglycol (PEG).

3. Non-covalent self-assembling hydrogel matrix according to claim 1 or 2, **characterised in that** the highly negatively charged oligosaccharide is a sulfated or phosphorylated oligosaccharide.

4. Non-covalent self-assembling hydrogel matrix according to claim 3, **characterised in that** the highly negatively charged oligosaccharide is selected from a group of oligosaccharides which comprises heparine, dextransulfate, α-cyclodextrinsulfate, β-cyclodextrinsulfate, γ-cyclodextrinsulfate, α-cyclodextrinphosphate, β-cyclodextrinphosphate and γ-cyclodextrinphosphate.

5. Non-covalent self-assembling hydrogel matrix according to one of claims 1 to 4, **characterised in that** it furthermore contains a chemical group that is light-cleavable between the polymer chain and the peptide sequence which includes the repeated dipeptide-motif (BA)n.

6. Non-covalent self-assembling hydrogel matrix according to one of claims 1 to 4, **characterised in that** it furthermore contains a pH sensitive chemical linker between polymer chain and peptide sequence which includes the repeating dipeptide-motif (BA)n.

7. Non-covalent self-assembling hydrogel matrix according to one of claims 1 to 4, **characterised in that** it furthermore comprises an enzymatic cleavable linker between the polymer chain and the peptide sequence which includes the repeating dipeptide-motif (BA)n.

8. Non-covalent self-assembling hydrogel matrix according to one of claims 1 to 7, **characterised in that** it exhibits an elasticity module of at least 10 Pa.

9. Non-covalent self-assembling hydrogel matrix according to one of claims 1 to 8, **characterised in that** it is spherically shaped.

10. Composite of a non-covalent self-assembling hydrogel matrix according to one of claims 1 to 9 with cells embedded in the spherical or non-spherical hydrogel matrix.

11. Composite according to claim 10, **characterised in that** the cells are selected from among a group which comprises mammalian cells, insect cells, bacteria cells and yeast cells.

12. Use of a composite according to claim 10 or 11 for producing a protein whereby the protein preferably comprises therapeutic monoclonal antibodies.

13. Capsule for the targeted release of therapeutic reagents, **characterised in that** the therapeutic reagents are encapsulated with the non-covalent self-assembling hydrogel matrix according to one of claims 1 to 8 or together with a spherical non-covalent self-assembling hydrogel matrix according to claim 9.

14. Combination of a non-covalent self-assembling hydrogel matrix according to one of claims 1 to 8 and therapeutic chemicals and reagents, **characterised in that** a gradient of chemicals and reagents is produced in the therapeutic hydrogel.

15. Hybrid system comprising a non-spherical non-covalent self-assembling hydrogel matrix according to one of claims 1 to 8 and a spherical non-covalent self-assembling hydrogel matrix according to claim 9, **characterised in that** the non-spherical non-covalent self-assembling hydrogel matrix and the spherical non-covalent self-assembling hydrogel matrix each exhibit a different chemical composition and a component of the hybrid system can be controlled by means of light, through selective chemical degradation or through enzymatic digestion.

## Revendications

1. Matrice d'hydrogel auto-assembla,te non covalente pour applications biotechnologiques, contenant un conjugué polymère-peptide covalent comprenant des conjugués d'au moins deux peptides couplés à une chaîne polymère et contenant la séquence peptidique d'un motif dipeptide répété (BA)n, B étant un acide aminé à chaîne latérale chargée positivement, A de l'alanine et n un nombre compris entre 5 et 20, **caractérisée en ce qu'**elle contient en outre un oligosaccharide chargé négativement, la matrice de l'hydrogel se présentant sous la forme d'un système oligosaccharide/peptide/polymère dans lequel le peptide est conjugué chimiquement au polymère, l'hydrogel étant obtenu par mélange du conjugué peptide-polymère et de l'oligosaccharide.

2. Matrice d'hydrogel auto-assemblante non covalente selon la revendication 1, **caractérisée en ce que** la chaîne polymère est constituée d'un polyéthylèneglycol (PEG) linéaire ou à plusieurs branches.

3. Matrice d'hydrogel auto-assemblante non covalente selon la revendication 1 ou 2, **caractérisée en ce que** l'oligosaccharide à forte charge négative est un oligosaccharide sulfaté ou phosphorylé.

4. Matrice d'hydrogel auto-assemblante non covalente selon la revendication 3, **caractérisée en ce que** l'oligosaccharide à forte charge négative est sélectionné dans un groupe d'oligosaccharides comprenant l'héparine, le sulfate de dextrane, le sulfate de a-cyclodextrine, le sulfate de β-cyclodextrine, le sulfate de y-cyclodextrine, le phosphate de a-cyclodextrine, le phosphate de β-cyclodextrine et le phosphate de y-cyclodextrine.

5. Matrice d'hydrogel auto-assemblante non covalente selon l'une des revendications 1 à 4, **caractérisée en ce qu'**elle englobe, en outre, un groupe chimique photoclivable entre la chaîne polymère et la séquence peptidique contenant le motif dipeptide répété (BA)ₙ.

6. Matrice d'hydrogel auto-assemblante non covalente selon l'une des revendications 1 à 4, **caractérisée en ce qu'**elle englobe, en outre, un lieur chimique sensible au pH entre la chaîne polymère et la séquence peptidique contenant le motif dipeptide répété (BA)ₙ.

7. Matrice d'hydrogel auto-assemblante non covalente selon l'une des revendications 1 à 4, **caractérisée en ce qu'**elle englobe, en outre, un lieur clivable par voie enzymatique entre la chaîne polymère et la séquence peptidique contenant le motif dipeptide répété (BA)n.

8. Matrice d'hydrogel auto-assemblante non covalente selon l'une des revendications 1 à 7, **caractérisée en ce qu'**elle présente un module d'élasticité d'au moins 10 Pa.

9. Matrice d'hydrogel auto-assemblante non covalente selon l'une des revendications 1 à 8, **caractérisée en ce qu'**elle présente une conformation sphérique.

10. Composite d'une matrice d'hydrogel auto-assemblante non covalente selon l'une des revendications 1 à 9 avec cellules incorporées dans la matrice d'hydrogel à conformation sphérique ou non-sphérique.

11. Composite selon la revendication 10, **caractérisé en ce que** les les cellules sont sélectionnées dans un groupe englobant les cellules de mammifères, les cellules d'insectes, les cellules de bactéries et les cellules de levure.

12. Utilisation d'un composite selon la revendication 10 ou 11 pour la production de protéines, ces dernières englobant des anticorps monoclonaux thérapeutiques.

13. Capsule pour la libération ciblée de réactifs thérapeutiques, **caractérisée en ce que** les réactifs thérapeutiques associés à une matrice d'hydrogel auto-assemblante non covalente selon l'une des revendications 1 à 8 ou à une matrice d'hydrogel auto-assemblante non covalente de conformation sphérique selon la revendication 9 sont encapsulés.

14. Composition à base d'une matrice d'hydrogel auto-assemblante non covalente selon l'une des revendications 1 à 8 et de produits chimiques et réactifs thérapeutiques, **caractérisée en ce qu'**un gradient des produits chimiques et des réactifs est généré dans l'hydrogel thérapeutique.

15. Système hybride issu d'une matrice d'hydrogel auto-assemblante non covalente de conformation non-sphérique selon les revendications 1 à 8 et d'une matrice d'hydrogel auto-assemblante non covalente de conformation sphérique selon la revendication 9, **caractérisé en ce que** la matrice d'hydrogel auto-assemblante non covalente de conformation non-sphérique et la matrice d'hydrogel auto-assemblante non covalente de conformation sphérique présentent une composition chimique distincte et **en ce qu'**une composante du système hybride est ajustable par irradiation de lumière, par dégradation chimique sélective ou par digestion enzymatique.
